# EUROPEAN PATENT SPECIFICATION

(11) **EP 3 990 020 B1**
(45) Date of publication and mention of the grant of the patent: **07.02.2024**
(21) Application number: 20733478.0
(22) Date of filing: 23.06.2020
(51) Int. Cl.: A61K 45/00, A61P 31/06

(54) **INHIBITOR OF SURFACE PROTEIN (SP-D) / SIRPA / SHP2 PATHWAY FOR USE IN THE PREVENTION AND/OR TREATMENT OF SECONDARY INFECTION**
INHIBITOR DES OBERFLÄCHENPROTEIN(SP-D)/SIRPA/SHP2-PFADES ZUR VERWENDUNG BEI DER VORBEUGUNG UND/ODER BEHANDLUNG VON SEKUNDÄRINFEKTIONEN
INHIBITEUR DE VOIE DE PROTÉINE DE SURFACE (SP-D)/SIRPA/SHP2 POUR UNE UTILISATION DANS LA PRÉVENTION ET/OU LE TRAITEMENT D'INFECTIONS SECONDAIRES

(30) Priority: 25.06.2019 EP 19305836
(43) Date of publication of application: 04.05.2022
(73) Proprietor: Nantes Université, 44035 Nantes Cedex 1 (FR); CHU de Nantes - Centre Hospitalier Universitaire de Nantes, 44000 Nantes (FR)
(72) Inventor: ROQUILLY, Antoine, 44300 NANTES (FR); ASEHNOUNE, Karim, 44000 NANTES (FR); JACQUELINE, Cédric, 44540 SAINT-MARS-LA-JAILLE (FR)
(74) Representative: Novagraaf Technologies
(86) International application number: PCT/EP2020/067498
(87) International publication number: WO 2020/260281

(56) References cited:
- WO-A2-2015/124928
- DELGADO CARLOS ET AL: "Serum Surfactant Protein D (SP-D) is a Prognostic Marker of Poor Outcome in Patients with A/H1N1 Virus Infection", LUNG, SPRINGER NEW YORK LLC, US, vol. 193, no. 1, 24 December 2014 (2014-12-24), pages 25-30, XP035442771, ISSN: 0341-2040, DOI: 10.1007/S00408-014-9669-3 [retrieved on 2014-12-24]
- WILLIAM J. JANSSEN ET AL: "Surfactant Proteins A and D Suppress Alveolar Macrophage Phagocytosis via Interaction with SIRP[alpha]", AMERICAN JOURNAL OF RESPIRATORY AND CRITICAL CARE MEDICINE., vol. 178, no. 2, 15 July 2008 (2008-07-15) , pages 158-167, XP055645371, US ISSN: 1073-449X, DOI: 10.1164/rccm.200711-1661OC
- LIN-XI LI ET AL: "Increased Susceptibility to Salmonella Infection in Signal Regulatory Protein [alpha]-Deficient Mice", THE JOURNAL OF IMMUNOLOGY, vol. 189, no. 5, 30 July 2012 (2012-07-30) , pages 2537-2544, XP055647470, US ISSN: 0022-1767, DOI: 10.4049/jimmunol.1200429 cited in the application
- HASSAN AHMED KHAN ET AL: "Nosocomial infections: Epidemiology, prevention, control and surveillance", ASIAN PACIFIC JOURNAL OF TROPICAL BIOMEDICINE, vol. 7, no. 5, 1 May 2017 (2017-05-01), pages 478-482, XP055647512, China ISSN: 2221-1691, DOI: 10.1016/j.apjtb.2017.01.019
- ROQUILLY ANTOINE ET AL: "Local Modulation of Antigen-Presenting Cell Development after Resolution of Pneumonia Induces Long-Term Susceptibility to Secondary Infections", IMMUNITY, CELL PRESS, US, vol. 47, no. 1, 18 July 2017 (2017-07-18), page 135, XP085134426, ISSN: 1074-7613, DOI: 10.1016/J.IMMUNI.2017.06.021

## Description

### FIELD OF THE INVENTION

The invention relates to inhibitor of surface protein D (SP-D) and/or inhibitor of SP-D / SIRPα interaction and/or SHP-2 for use in the prevention and/or the treatment of secondary disease, in particular nosocomial disease.

The present invention also relates to pharmaceutical composition comprising Inhibitor of surface protein D (SP-D) and/or Inhibitor of SP-D - SIRPα interaction and/or inhibitor of SHP-2 for use in the prevention and/or the treatment of secondary disease, in particular nosocomial disease.

The present invention finds application in the therapeutic and diagnostic medical technical fields.

### BACKGROUND OF THE INVENTION

Alveolar macrophages (AM) monitor the luminal surface of the epithelium where air-borne bacteria grow and, together with epithelial cells, contribute to set the threshold and the quality of the innate immune response in the lung mucosa 13.

Healthy lungs are colonized by bacteria whose burden is continuously controlled by mucosal immunity (Charlson et al., 2011 [1]). Infection by pathogenic bacteria disrupts this balance and can induce lung injury through direct damage caused by the pathogen, or through immunopathology elicited by the effector mechanisms of immunity. Therefore, a healthy immune response should maximize the deployment of effector mechanisms against the pathogen while minimizing the damage of self-tissues that may ensue.

It is well known that Nosocomial infections (NI) increase morbidity and mortality. In particular, the most common NI are surgical site infections, infections of the gastrointestinal tract and respiratory tract, urinary tract infections, and primary sepsis. (Ella Ott, Dr. med., et al. "The Prevalence of Nosocomial and Community Acquired Infections in a University Hospital An Observational Study Dtsch Arztebl Int. 2013 Aug; 110(31-32): 533-540 [2]).

Pneumonia is the leading cause of death from infectious disease (Mizgerd, 2006 [3]). The risk of developing pneumonia increases following severe primary infections and reaches 30-50% for critically ill patients recovering from a first episode of infection (van Vught et al., 2016a [59]). It is currently accepted that susceptibility to secondary pneumonia increases due to acquired immune defects collectively known as sepsis-induced immunosuppression (Hotchkiss et al., 2013a [4]; Roquilly and Villadangos, 2015 [5]). In-depth understanding of the mechanisms involved is vital to prevent and treat secondary pneumonia in patients recovering from a primary infection.

Hospital-acquired pneumonia (HAP) is the most frequent form of hospital-acquired infection, with 500,000 episodes reported every year in Europe. HAP has an attributable mortality rate of 10%, requires prolonged hospitalization, and reduces the patient's quality of life after release (Bekaert, M. et al. [6], GBD 2015 DALYs and HALE Collaborators [7]). The European Centre for Disease Prevention and Control reported that respiratory tract infections are responsible for 33% of antimicrobial use in European hospitals. Since HAP is frequently induced by drug-resistant pathogens it is a major cause of broad-spectrum antibiotic consumption in intensive care units, which in turn promotes antibiotic resistance. HAP also imposes a high economic burden on the public health system, as the average cost per episode exceeds €40,000. In the United States, HAP management costs $8 billion a year (Eber, M. R., et al. 2010 [8]). Despite the development of international recommendations (Torres, A. et al. (2017) [9], Kalil, A. C. et al 2016 [10]), therapies and preventive measures aimed at reducing the bacterial burden as a strategy to prevent HAP have not led to improved outcomes, and treatment failures are still common (Klompas, M. 2009 [11], Weiss, E. 2017 [12]). A better understanding of the factors that influence HAP onset is urgently needed to develop innovative and more efficient therapies. Of particular interest are strategies aimed at predicting susceptibility to nosocomial disease, such as HAP and improving the resistance of the host to infection.

The risk of developing secondary infection, in particular nosocomial infection, such as HAP reaches 30-50% in critically ill patients recovering from a critical medical condition (Asehnoune, K. et al. 2014 [13], Van Vught, L. A. et al. 2016 [14]). The traditional explanation for the increase in susceptibility has been that the lungs of these patients become colonized by bacteria that do not normally access the lower respiratory tract, causing infection. An alternative explanation for the increased susceptibility of critically-ill patients to HAP has thus emerged, namely that they are less capable of controlling infection. Support for this hypothesis has been obtained from mouse and clinical studies showing that sepsis, severe trauma and other triggers of systemic inflammation cause immune defects collectively known as critical illness-related immunosuppression (Belkaid, Y. & Harrison, O. J. 2017 [15], Charlson, E. S. et al, 2011 [16]). The specific mechanisms of immunosuppression have only started to be unraveled. Dendritic cells (DC) have been shown to contribute to control bacterial infection and that sepsis or trauma causes impairment of their function (paralysis) (Hotchkiss, R. S et al. 2013 [17]). DC paralysis may have an effect in the immunosuppression in the lung, however immunosuppression, in particular in the lung is not due to Dendritic cells impairment. It is known that a person having an immunosuppression is more susceptible to infection, in particular bacterial infection, and that, at the hospital, immunocompromised person are more susceptible to Nosocomial Infection. In addition, it is well known that Nosocomial infection, when due to bacterial infection, are strong infection which are, in most of the time, resistant to the most common antibiotic compound. Thus, these therapies have to be improved since they do not allow to effectively treat the NI and/or are less effective in the treatment than expected.

There is therefore a real need to find a method and/or a compound which could prevent nosocomial infection and/or suppress / decrease the immunosuppression, in particular illness-related immunosuppression.

There is therefore a real need to find a method and/or a compound which allows more efficient treatment and/or effective treatment of Nosocomial Infections (NI). In particular there is a real need to find new strategies, i.e. new targets/pathways, in the treatment Nosocomial infections (NI).

### Description of the invention

The present invention meets these needs and overcomes the abovementioned drawbacks of the prior art with the use of inhibitor of surface protein D (SP-D) and/or inhibitor of SP-D-SIRPα interaction and/or inhibition of the activation of SHP-2 by SIRPα for the prevention and/or treatment of secondary disease, in particular nosocomial disease.

The inventors have demonstrated that the functional properties of AM before, during and after resolution of pneumonia in mice, which are similar to human AM and monocytes, showed profound functional defects for months after resolution of infection. The most salient defect was poor capacity to phagocytose bacteria, for example due to the modulation of the cellular microenvironment by SIRP-α stimulation.

The inventors have also demonstrated that the functional properties of AM before, during and after resolution of septic or non-septic inflammation in mice, which are similar to human AM and monocytes, showed profound functional defects for months after resolution of infection. The most salient defect was poor capacity to phagocytose bacteria, for example due to the modulation of the cellular microenvironment by SIRP-α stimulation.

In particular, the macrophages and dendritic cells (DC) orchestrate immunity and tolerance, the inventors have compared their functional properties before, during and after resolution of a first infection, for example pneumonia, and demonstrated that both cell types showed profound alterations, also mentioned herewith as "paralysis". Paralysis was caused by the SIRP-A dependent modulation of local mediators involved in immune homeostasis. The inventors have supported that DC and macrophage dysfunction is an important contributor to protracted immunosuppression after bacterial or viral primary sepsis and increased susceptibility to secondary infection, for example Nosocomial Infections (NI) such as a secondary pneumonia.

In addition, the inventors have compared macrophages and dendritic cells (DC) functional properties before, during and after resolution of a first inflammation, for example pneumonia or trauma, and demonstrated that both cell types showed profound alterations, also mentioned herewith as "paralysis". Paralysis was caused by the SIRP-A dependent modulation of local mediators involved in immune homeostasis. The inventors have supported that DC and macrophage dysfunction is an important contributor to protracted immunosuppression after bacterial or viral primary sepsis and increased susceptibility to secondary infection, for example Nosocomial Infections (NI) such as a secondary pneumonia.

Sepsis and trauma cause inflammation-induced immunosuppression and elevated susceptibility to secondary infection, for example hospital-acquired pneumonia. The inventors have also surprisingly demonstrated that after resolution of primary bacterial or viral pneumonia, alveolar macrophages (AM) exhibited poor phagocytic capacity for several weeks. The inventors have also surprisingly demonstrated that after resolution of primary bacterial or viral pneumonia or non-septic lung inflammation, alveolar macrophages (AM) exhibited poor phagocytic capacity for several weeks. The inventors have also demonstrated that the "paralyzed" AM have been developed from resident AM with a transcriptional programming driving molecular functions of cytokine receptor activity and tyrosine kinase activity. The reprogramming of the newly formed AM was induced locally by immunosuppressive signals established upon resolution of primary infection, not by direct encounter of the pathogen. The inventors have also demonstrated that the tyrosine kinase-inhibitory receptor, SIRP-α, played a critical role in the modulation of the suppressive microenvironment. The inventors have also demonstrated that in human suffering systemic inflammation, not only AM but also circulating monocytes displayed phenotypical alterations for up to six months after resolution of inflammation, and that these reprogramming is associated with the risk of hospital-acquired pneumonia.

The inventors have also demonstrated that the variation of SP-D concentration is inversely correlated with the variation of phagocytotic AM. In other words, the inventors have surprisingly demonstrated that an increase of SP-D concentration is associated with a protracted decrease of phagocytosis by AM, and that this defect lasts after the normalization of the SP-D concentration, in particular because SIRP-α activation alters for weeks the cellular microenvironment.

The inventors have demonstrated that the alteration of immune cells may, in part, be responsible of the increase of susceptibility of human to secondary infection after, for example inflammation, sepsis and/or trauma and/or major surgery.

The inventors have also demonstrated that the use of inhibitor of SP-D / SIRPα interaction allows to treat secondary infection, for example Nosocomial infections whatever is the Nosocomial infection. In other words, the inventors have demonstrated that inhibitor of activation of SIRPA by surface protein D (SP-D) allows to treat and/or to prevent Nosocomial infections and also to inhibit protracted immunosuppression after, for example bacterial and/or viral and/or fungus primary sepsis and/or infections and/or trauma and/or acute inflammation and/or major surgery.

The inventors have also demonstrated that inhibitor of surface protein D (SP-D) and/or inhibitor of SP-D / SIRPα interaction allows to treat and/or to prevent Nosocomial infections and also to inhibit protracted immunosuppression after, for example bacterial and/or viral and/or fungus primary sepsis and/or infections and/or trauma and/or acute inflammation and/or major surgery.

In addition, the inventors have also demonstrated that inhibitor of surface protein D (SP-D) allows to prevent secondary infections in a systemic way. In other words, the inventors have demonstrated that after a primary condition, for example bacterial and/or viral and/or fungus primary sepsis and/or infections and/or after conditions that could induce primary inflammation, for example trauma, hemorrhage, infection, major surgery; inhibitor of the interactions between protein D (SP-D) and SIRPA restores phagocytosis by circulating monocytes and allows to prevent secondary infection whatever the localization and/or the organ infected. In particular, the inventors have demonstrated unexpectedly that inhibitor of SIRPA / surfactant protein D (SP-D) interaction provides a systemic protection which advantageously would allow to prevent and/or treat a secondary infection which could appear at different localization and/or in different organ with regards to the primary infection.

In addition, the inventors have also demonstrated that inhibitor of SHP-2, in particular inhibition of the activation of SHP-2 by SIRPα allows to prevent secondary infections in a systemic way. In other words, the inventors have demonstrated that after a primary condition, for example bacterial and/or viral and/or fungus primary sepsis and/or infections and/or after conditions that could induce primary inflammation, for example trauma, hemorrhage, infection, major surgery; inhibitor of SHP-2, in particular inhibition of the activation of SHP-2 by SIRPα restores phagocytosis by circulating monocytes and allows to prevent secondary infection whatever the localization and/or the organ infected. In particular, the inventors have demonstrated unexpectedly that inhibitor of inhibition of the activation of SHP-2 by SIRPα provides a systemic protection which advantageously would allow to prevent and/or treat a secondary infection which could appear at different localization and/or in different organ with regards to the primary infection.

Moreover, the inventors have demonstrated that the present invention allows surprisingly and unexpectedly to prevent and/or treat secondary infections whatever the cause of the secondary infections. In other words, the origin and/or cause of the secondary infection may be advantageously different from the origin and/or cause of the primary infection.

An object of the present invention is an inhibitor of surface protein D (SP-D) for use in the prevention and/or the treatment of secondary infection, wherein the inhibitor of surface protein (SP-D) is selected from anti-SP-D antibody, an anti-SP-D antibody fragment, a recombinant anti-SP-D antibody, a binding peptide, a siRNA, an antisense oligo or a ligand trap.

In the present surface protein D (SP-D) means any surface protein D (SP-D or SFTPD) known to one skilled in the art. It may be for example the surface protein D (SP-D) disclosed in Kishore U, Greenhough TJ, Waters P, Shrive AK, Ghai R, Kamran MF, et al. (2006). "Surfactant proteins SP-A and SP-D: structure, function and receptors". Mol Immunol. 43 (9): 1293-315. doi:10.1016/j.molimm.2005.08.004. PMID 16213021 [18]. It may be for example the human surface protein D (SP-D) as disclosed in Jens Madsen, Anette Kliem, Ida Tornrae, Karsten Skjradt, Claus Koch and Uffe Holmskov. Localization of Lung Surfactant Protein D on Mucosal Surfaces in Human Tissues. J Immunol 2000; 164:5866-5870; doi: 10.4049/jimmunol.164.11.5866. PMID:10820266 [19] and/or of sequence with Protein Accession number P35247.3.

In the present inhibitor of surface protein D (SP-D) may be any inhibitor of surface protein D (SP-D) known from one skilled in the art. It may be for example an inhibitor of surface protein D (SP-D) expression, an inhibitor of surface protein D (SP-D). It may be for example an anti-SP-D antibody, an anti-SP-D antibody fragment, a recombinant anti- SP-D antibody, a binding peptide, a siRNA, an antisense oligo, a ligand trap.

It may be for example a commercially available anti-SP-D antibody adapted for the treatment of human being. It may be for example a commercially available inhibitor of surface protein D (SP-D), for example an anti-SP-D antibody, for example commercialized under the reference MOB-1777z by creative lab, Anti-Surfactant protein D/SP-D antibody commercialized under the reference ab203309 or ab97849 by abcam.

It may be for example antibody of any mammal origin adapted for the treatment of human being. It may be for example, antibodies obtained according to the process disclosed in Leffleur et al. 2012 [20] comprising administering 1 ng to 100 mg of anti-SP-D beta antibody.

In the present antibodies against SP-D may be mouse antibody, for example any mouse antibody known from one skilled in the art that could inhibit SP-D. It may be for example a commercially available mouse antibody.

In the present antibodies against SP-D may be rabbit antibody, for example any rabbit antibody known from one skilled in the art that could inhibit SP-D. It may be for example a commercially available rabbit antibody, for example rabbit antibody referenced ab97849 commercialized by abeam.

In the present antisense oligo may be any corresponding antisense oligo known from one skilled in the art that could inhibit SP-D. It may be for example a commercially available antisense oligo, for example EPI-2010 (Makoto Tanaka and Jonathan W Nyce. Respirable antisense oligonucleotides: a new drug class for respiratory disease. Respiratory Research 2000 2:2. https://doi.org/10.1186/rr32 [21]). In the present peptide may be any peptide known from one skilled in the art that could inhibit SP-D. It may be for example a commercially available peptides, for example peptide referenced LS-E15283 commercialized by LSBio.

In the present ligand trap may be any ligand trap known from one skilled in the art that could inhibit SP-D. It may be for example a commercially available ligand trap.

In the present small molecules may be any small molecules known from one skilled in the art that could inhibit SP-D. It may be for example a commercially available small molecules.

In the present inhibitor of SP-D synthesis may be inhibitor of SP-D synthesis known from one skilled in the art. It may be for example a commercially available inhibitor of SP-D synthesis.

Advantageously, the inhibitor of surface protein D (SP-D) may be anti-SP-D antibody, an anti-SP-D antibody fragment, a recombinant anti-SP-D antibody, a binding peptide, in particular anti-SP-D antibody, an anti-SP-D antibody fragment, an anti- SP-D binding peptide.

According to the invention, Inhibitors of SP-D may be administered on a single administration or repeated administration, for example one to three time per day, for example for a period up to 28 days.

Inhibitors of SP-D may be administered to humans and other animals orally, rectally, parenterally, intracisternally, intravaginally, intraperitoneally, topically (as by powders, ointments or drops), buccally, as oral or nasal spray, subcutaneously, or the like, depending on the severity of the infection to be treated.

The way of administration of the inhibitor of surface protein D (SP-D) may be adapted with regards to the inhibitor used. One skilled in the art taking into consideration his technical knowledge would adapt the administration way to the used inhibitor.

The doses of inhibitors surface protein D (SP-D) to be administered may be adapted with regards to the inhibitor used. One skilled in the art taking into consideration his technical knowledge would adapt the administered doses to the used inhibitor.

For example, the inhibitor of surface protein D (SP-D) may be administered at doses of from about 0.1 to 2000 µg, for example at doses of from about 0.1 to 1000 µg
For example, when the inhibitors of surface protein D (SP-D) is antibodies, for example commercialized under the reference MOB-1777z by creative lab, Anti-Surfactant protein D/SP-D antibody commercialized under the reference ab203309 or ab97849 by abcam, it may be administered, for example at doses around 1 mg. For example, when the inhibitors of the inhibitor of surface protein D (SP-D) is recombinant protein, it may be administered, for example at doses from 0.1 to 2000 µg, for example at doses from 0.1 to 1000 µg, preferably from 100 to 1000µg. For example, when the inhibitors of the inhibitor of surface protein D (SP-D) is humanized antibody, it may be administered, for example at doses from 0.1 to 100 µg.

For example, inhibitors of surface protein D (SP-D) may be administered at a dose from 0.01 µg/kg to 10 µg/Kg body weight of the subject per day, one or more times a day, to achieve the desired therapeutic effect. Inhibitors of surface protein D (SP-D) may also be administered at a dose from 0.01 µg/kg to 20 µg/Kg body weight of the subject per day, one or more times a day, to achieve the desired therapeutic effect.

An object of the present invention is an inhibitor of SP-D / SIRPα interaction for use in the prevention and/or the treatment of secondary infection, wherein the inhibitor of SP-D/SIRPα interaction is selected from an anti-SIRPα antibody, an anti- SIRPα antibody fragment, a recombinant anti-SIRPα antibody, a binding peptide, a siRNA, an antisense oligo, a ligand trap or a small molecule SIRPα inhibitor.

In the present SP-D / SIRPα interaction means any interaction that could appears between SP-D and SIRPα known from one skilled in the art. It may be for example hydrophobic interaction, hydrogen interaction, covalent binding, ligand-receptor binding, ionic interaction, hydrophobic bonding, van der Waals forces, salt bridges.

In the present inhibitors of SP-D / SIRPα interaction may be any inhibitors known from one skilled in the art adapted to inhibit the SP-D / SIRPα interaction. It may be for example inhibitor of SP-D and/or of SIRPα.

In the present inhibitors of the SP-D / SIRPα interaction may be, for example an inhibitor of surface protein D (SP-D) expression, an inhibitor of surface protein D (SP-D). The inhibitor of SP-D may be as defined above. It may be for example an anti-SP-D antibody, an anti-SP-D antibody fragment, a recombinant anti-SP-D antibody a binding peptide, a siRNA, an antisense oligo, a ligand trap.

In the present inhibitors of the SP-D / SIRPα interaction may be, for example an inhibitor of SIRPα expression, an anti-SIRPα antibody, an anti-SIRPα antibody fragment, a recombinant anti-SIRPα antibody, a binding peptide, a siRNA, an antisense oligo, a ligand trap. It may be for example antibodies against SIRPα, antisense oligo, peptides, mouse antibody, ligand trap, small molecules, pyrrole- imidazole polyamide, inhibitor of SIRPα synthesis, humanized antibody.

It may be for example a commercially available anti-SIRPα antibody adapted for the treatment of human being. It may be for example a commercially available inhibitor of SIRPα, for example an anti-SIRPα antibody, for example commercialized under the reference Clone P84 commercialized by Fisher scientific, under the reference ab53721 commercialized by abcam.

Anti-SIRPα antibody may be for example antibody of any mammal origin adapted for the treatment of human being. It may be for example, antibodies obtained according to the process disclosed in Leffleur et al. 2012 [20] comprising administering of anti-SIRPα antibody.

In the present antibodies against SIRPα may be mouse antibody, for example any mouse antibody known from one skilled in the art that could inhibit SIRPα. It may be for example a commercially available mouse antibody, for example mouse antibody referenced CBL650 commercialized by Creative biolabs.

In the present antibodies against SIRPα may be a rabbit antibody, for example any rabbit antibody known from one skilled in the art that could inhibit SIRPα. It may be for example a commercially available rabbit antibody, for example rabbit antibody referenced ab53721 commercialized by abcam.

In the present antisense oligo inhibitor of SIRPα may be any corresponding antisense oligo known from one skilled in the art that could inhibit SIRPα. It may be for example a commercially available antisense oligo that could inhibit SIRPα.

In the present inhibitor peptide of SIRPα may be any peptide known from one skilled in the art that could inhibit SIRPα. It may be for example a commercially available peptides that inhibit SIRPα.

In the present ligand trap inhibitor of SIRPα may be any ligand trap known from one skilled in the art that could inhibit SIRPα. It may be for example a commercially available ligand trap inhibitor of SIRPα.

In the present small molecules inhibitor of SIRPα may be any small molecules known from one skilled in the art that could inhibit SIRPα. It may be for example a commercially available small molecules inhibitor of SIRPα, for example small molecules referenced NSC-87877 (Rongjun He, Li-Fan Zeng, Yantao He, Sheng Zhang, Zhong-Yin Zhang. Small molecule tools for functional interrogation of protein tyrosine phosphatases FEBS Journal 2013 https://dx.doi.org/10.1111/j. 1742-4658.2012.08718.x [22]).

Advantageously, the inhibitor of SIRPα may be anti-SIRPα antibody, an anti-SIRPα antibody fragment, a recombinant anti- SIRPα antibody, a binding peptide, in particular anti-SIRPα antibody, an anti-SIRPα antibody fragment, an anti- SIRPα binding peptide.

In the present inhibitors of SIRPα may also be, for example a modulator, for example a suppressor or an activator, of the intracellular pathways, and/or biological process and/or molecular function and/or phenotype and/or gene expression and/or protein which may be controlled and/or regulated by SIRPα, are mentioned in the present as inhibitors of SIRPα pathway.

Inhibitors of SIRPα pathway may be for example a modulator, for example a suppressor or an activator, of the biological process which may be controlled and/or regulated by SIRPα, for example a modulator of the chemokine-mediated signaling pathway, a modulator of the leukocyte migration, a modulator of the inflammatory response. It may be for example a modulator of the expression of genes selected from the group comprising 1810011010Rik, 4930502E18Rik, Areg, Arg1, Bambi-ps1, Ccl3, Ccl4, Ccl7, Ccrl2, Cd276, Cdc25c, Clec1b, Cmbl; Col4a1, Col4a2, Cxcl1, Cxcl10, Cxcl2, Cxcl3, Ddah1, Dusp2, Ecm1, Ereg, Esrrg, Exd1, Fads2, Fam198b, Fam46c, Gadd45a, Gadd45b, Gatm, Gchfr, Gm6377, Has1, Hey1, Hmcn1, Id3, Il6, Kcnk13, Lypd6b, Mfge8, Nfkbiz, Pdk4, Ptgs2, Ptpn11, Rasef, Rgcc, Rgs1, Rhov, Scd1, Socs3, Syt3, Tnf, Tnfaip3, Ttll7, Xist, OaS2, D1Pas1 or Mir142. It may be for example a modulator, in particular an activator of the expression of genes selected from the group comprising 1810011O10Rik, 4930502E18Rik, Areg, Arg1, Bambi-ps1, Ccl3, Ccl4, Ccl7, Ccrl2, Cd276, Cdc25c, Clec1b, Cmbl; Col4a1, Col4a2, Cxcl1, Cxcl10, Cxcl2, Cxcl3, Ddah1, Dusp2, Ecm1, Ereg, Esrrg, Exd1, Fads2, Fam198b, Fam46c, Gadd45a, Gadd45b, Gatm, Gchfr, Gm6377, Has1, Hey1, Hmcn1, Id3, Il6, Kcnk13, Lypd6b, Mfge8, Nfkbiz, Pdk4, Ptgs2, Rasef, Rgcc, Rgs1, Rhov, Scd1, Socs3, Syt3, Tnf, Tnfaip3, Ttll7, Xist. It may be for example a modulator, in particular a suppressor of the expression of genes selected from the group comprising OaS2, D1Pas1, Ptpn11 or Mir142.

Inhibitors of SP-D - SIRPα interaction may be administered to humans and other animals orally, rectally, parenterally, intracisternally, intravaginally, intraperitoneally, topically (as by powders, ointments or drops), buccally, as oral or nasal spray, subcutaneously, by aerosol or the like, depending on the severity of the infection to be treated.

The way of administration of inhibitors SP-D - SIRPα interaction may be adapted with regards to the inhibitor used. One skilled in the art taking into consideration his technical knowledge would adapt the administration way to the used inhibitor.

The way of administration of the inhibitor of inhibitors of SP-D - SIRPα interaction may be adapted with regards to the inhibitor used. One skilled in the art taking into consideration his technical knowledge would adapt the administration way to the used inhibitor.

According to the invention, inhibitors of SP-D - SIRPα interaction may be administered on a single time or repeated administration, for example one to three time per day, for example for a period up to 28 days.

The doses of inhibitors of SP-D/SIRPα interaction to be administered may be adapted with regards to the inhibitor used. One skilled in the art taking into consideration his technical knowledge would adapt the administered doses to the used inhibitor.

For example, the inhibitor of SP-D - SIRPα interaction may be administered at doses from about 0.01 to 2000 µg, for example from about 0.01 to1000 µg.

An object of the present invention is also an inhibitor of SHP-2 for use in the prevention and/or the treatment of secondary infection, wherein the inhibitor of SHP-2 is selected from an anti-SIRPα antibody, an anti- SIRPα antibody fragment, a recombinant anti-SIRPα antibody, a binding peptide, a siRNA, an antisense oligo, a ligand trap or a small molecule SIRPα inhibitor.

In the present SHP-2 refers to the sph2 Gene and/or the protein encoded by sph2 Gene also known as PTPN11 (protein tyrosine phosphatase non-receptor type 11) gene. SHP-2 is also known as being activated by SIRPα
In the present inhibitor of SHP-2 may be any inhibitor of SHP-2 known from one skilled in the art. It may be for example an inhibitor of the expression of SHP-2 gene and/or an inhibitor of the protein encoded by the SHP-2 gene.

Inhibitor of SHP-2 may be for example a binding peptide, a siRNA, an antisense oligo, a ligand trap, a small molecule, an antibody.

In the present small molecules that inhibit SHP 2 may be any small molecules known from one skilled in the art It may be for example a commercially available small molecules, for example small molecules referenced small-molecule SHP2 inhibitor, SHP099, a small molecule disclosed in Ying-Nan P. Chen, Matthew J. LaMarche, Ho Man Chan, et al. Allosteric inhibition of SHP2 phosphatase inhibits cancers driven by receptor tyrosine kinases. Nature 2016, https://dx.doi.org/10.1038/nature18621 [23]) or G493 disclosed in Linxiang Lan, Jane D Holland, Jingjing Qi, Stefanie Grosskopf, Regina Vogel, Balázs Györffy, Annika Wulf-Goldenberg, Walter Birchmeier Shp2 signaling suppresses senescence in PyMT-induced mammary gland cancer in mice The EMBO Journal 2015, 10.15252/embj.201489004 [47].

In the present inhibitor of SHP-2 may be inhibitor of SIRPα which inhibit of the activation of SHP-2 (PTPN11 gene) by SIRPα. It may be for example Protein-based biosensors targeting the SIRPα signaling pathway such as SIRPα Syk-iSNAP disclosed by Sun, Lei, Wang et al. (Nature Communications, Engineered proteins with sensing and activating modules for automated reprogramming of cellular functions 2017 https://dx.doi.org/10.1038/s41467-017-00569-6 [24)
Inhibitors of SHP-2 may be administered to humans and other animals orally, rectally, parenterally, intracisternally, intravaginally, intraperitoneally, topically (as by powders, ointments or drops), buccally, as oral or nasal spray, subcutaneously, by aerosol or the like, for example depending on the severity of the infection to be treated.

The way of administration of inhibitors SHP2 pathway may be adapted with regards to the inhibitor used. One skilled in the art taking into consideration his technical knowledge would adapt the administration way to the used inhibitor. The way of administration of the inhibitor of SHP-2 pathway may be adapted with regards to the inhibitor used. One skilled in the art taking into consideration his technical knowledge would adapt the administration way to the used inhibitor.

According to the invention, inhibitors of SHP-2 may be administered on a single time or repeated administration, for example one to three time per day, for example for a period up to 28 days.

The doses of inhibitors of SHP-2 to be administered may be adapted with regards to the inhibitor used. One skilled in the art taking into consideration his technical knowledge would adapt the administered doses to the used inhibitor. For example, the inhibitor of SHP-2 may be administered at doses of from about 0.01 to 2000 mg. For example when the inhibitor of SHP-2 is G493, it may be may be administered at doses of from about 0.01 to 2000 mg. For example, the inhibitor of SHP-2 may be administered at doses of from about 0.01 to 1000 mg. For example when the inhibitor of SHP-2 is G493, it may be may be administered at doses of from about 0.01 to 1000 mg.

In the present secondary infection means any infection which may occur after a primary infection and/or inflammation and/or postoperatively. It may be for example an infection occurring 1 to 90 days after the beginning of a primary infection, for example 5 to 12 day after the beginning of a primary infection. It may be also for example an infection occurring 1 to 90 days after the end of a primary infection for example 5 to 12 day after the end of the primary infection and/or the absence of any pathological sign and/or symptom.

In the present the secondary infection may be for example the origin and/or cause of the secondary infection may be advantageously different from the origin and/or cause of the primary infection.

In the present the secondary infection may for example affect other organ or another part of the subject compares to the primary infection, and/or inflammation. In other words, the secondary infection may affect an organ and/or part of the body which is different from the organ and/or part of the body infected by the primary infection and/or inflammation.

In the present the secondary infection may be any infection occurring after a primary infection known to one skilled in the art. It may be for example any secondary infection of gastrointestinal tract, respiratory tract, urinary tract infections. It may be for example any secondary infection of organ selected for the group comprising lung, blood, liver, eye, heart, breast, bone, bone marrow, brain, meninges, mouth, head & neck, esophageal, tracheal, stomach, colon, pancreatic, cervical, uterine, bladder, prostate, testicular, skin, rectal, lymphomas.

In the present the secondary infection may be a secondary infection selected from the group comprising pneumonia, pleural infection, urinary infection, peritoneal infection, intra-abdominal abscess, meningitis, mediastinal infection, soft-tissue or skin infection, such as cellulitis or surgical site infections, for example including bone, peritoneum, mediastinum, meninges, prothetic infections. For example it may be a secondary infection selected from the group comprising pneumonia, pleural infection, urinary infection, peritoneal infection, intra-abdominal abscess, meningitis and mediastinal infection.

In the present, the secondary infection may be due to any pathogen known to one skilled in the art. The secondary infection may be due to a bacteria selected from the group comprising *Staphylococcus aureus, Methicillin resistant Staphylococcus aureus, Streptococcus pneumoniae, Pseudomonas aeruginosa, Enterobacter spp (including E. cloacae), Acinetobacter baumannii, Citrobacter spp (including C. freundii, C. koserii), Klebsiella spp (including K. oxytoca, K. pneumoniae), Stenotrophomonas maltophilia, Clostridium difficile, Escherichia coli, Heamophilus influenza, Tuberculosis, Vancomycin-resistant Enterococcus, Legionella pneumophila. Other types include L. longbeachae, L. feeleii, L. micdadei, and L. anisa.*

In the present, the secondary infection may be due to any virus known to one skilled in the art. It may be for example any virus mentioned in CELIA AITKEN et al. "Nosocomial Spread of Viral Disease" Clin Microbiol Rev. 2001 Jul; 14(3): 528-546 [25]. It may be due to a virus selected from the group comprising RSV, influenza viruses A and B, parainfluenza viruses 1 to 3, rhinoviruses, adenoviruses, measles virus, mumps virus, rubella virus, parvovirus B19, rotavirus, enterovirus, hepatitis A virus, hepatitis B virus, hepatitis C virus, herpes simplex virus (HSV) types 1 and 2, Varicella-Zoster Virus (VZV), Cytomegalovirus (CMV), Epstein Barr virus (EBV), and human herpesviruses (HHVs) 6, 7, and 8, Ebola virus, Marburg virus, Lassa fever virus, Congo Crimean hemorrhagic fever virus, Rabies virus, Polyomavirus (BK virus).

In the present, the secondary infection may be due to any fungus known to one skilled in the art. It may be for example any fungus disclosed in SCOTT K. FRIDKIN et al. "Epidemiology of Nosocomial Fungal Infection" Clin Microbiol Rev, 1996 ; 9(4): 499-511 [26]. The secondary infection may be due to a specie of fungus selected from the group comprising *Candida spp, Aspergillus spp, Mucor, Adsidia, Rhizopus, Malassezia, Trichosporon, Fusarium spp, Acremonium, Paecilomyces, Pseudallescheria.*

In the present the secondary infection may be a nosocomial infection. It may be a nosocomial infection of any organ as previously mentioned. It may be a nosocomial infection due to any pathogen selected from the group comprising virus, bacteria and fungus. It may be a nosocomial infection due to a virus as previously defined. It may be a nosocomial infection due to a bacteria as previously defined. It may be a nosocomial infection due to a fungus as previously defined. It may be nosocomial infection selected from the group comprising pneumonia, pleural infection, urinary infection, peritoneal infection, intra-abdominal abscess, meningitis, mediastinal infection, or surgical site infections, for example on bone, peritoneum, mediastinum, meninges).. It may be nosocomial infection selected from the group comprising pneumonia, pleural infection, urinary infection, peritoneal infection, intra-abdominal abscess, meningitis, mediastinal infection, soft-tissue or skin infection (cellulitis), head & neck infection (including otitis).

The secondary infection may be a nosocomial infection, in particular pneumonia.

The secondary infection may be a nosocomial infection, for example an infection originated from hospital and/or acquired at the hospital and/or hospital-acquired infection.

In a particular embodiment, the secondary infection may be secondary pneumonia and/or a hospital-acquired pneumonia.

In the present primary infection means an infection due to any pathogen, or sepsis-like syndrome, that could have a negative effect on immune response and/or induce an immunosuppression.

In the present primary infection means an infection due to a pathogen selected from the group comprising bacteria, virus or fungus. It may be for example any infection due to pathogen selected from the group comprising bacteria, virus or fungus known from one skilled in the art. It may be for example an infection of gastrointestinal tract, respiratory tract, urinary tract infections, and primary sepsis. It may be for example any infection due to a pathogen selected from the group comprising virus, bacteria and fungus. It may be for example a non-documented infection, for example an infection wherein no pathogens have been searched or found, such as sepsis-like syndrome. In the present the primary infection may be any infection due to a pathogen of at least one organ selected for the group comprising lung, liver, eye, heart, breast, bone, bone marrow, brain, head and neck, esophageal, tracheal, stomach, colon, pancreatic, cervical, uterine, bladder, prostate, testicular, skin, rectal, and lymphomas.

Another object of the present invention is a pharmaceutical composition comprising inhibitors of SP-D thereof and a pharmaceutically acceptable carrier.

Another object of the present invention is a pharmaceutical composition comprising inhibitor of SP-D and/or inhibitor of SP-D - SIRPα interaction and/or inhibitor of SHP-2 and a pharmaceutically acceptable carrier for use in the prevention and/or treatment of a secondary infection, wherein the inhibitor of SP-D, the inhibitor of SP-D - SIRPα interaction and the inhibitor of SHP-2 is as defined above.

The pharmaceutical composition may be in any form that can be administered to a human or an animal. The person skilled in the art clearly understands that the term "form" as used herein refers to the pharmaceutical formulation of the medicament for its practical use. For example, the medicament may be in a form selected from the group comprising an injectable form, aerosols forms, an oral suspension, a pellet, a powder, granules or topical form, for example cream, lotion, collyrium, sprayable composition.

As described above, the pharmaceutically acceptable compositions for use according to the present invention further comprise a pharmaceutically acceptable carrier, adjuvant or carrier. The pharmaceutically acceptable carrier may be any known pharmaceutical support used for the administration to a human or animal, depending on the subject to be treated. It may be any solvent, diluent or other liquid carrier, dispersion or suspension, surfactant, isotonic agent, thickening or emulsifying agent, preservative, solid binder, lubricant and the like, adapted to the particular desired dosage form. Remington Pharmaceutical Sciences, sixteenth edition, E. W. Martin (Mack Publishing Co., Easton, Pa., 1980) [27] discloses various carriers used in the formulation of pharmaceutically acceptable compositions and known techniques for their preparation. Except in the case where a conventional carrier medium proves incompatible with the compounds for use according to the invention, for example by producing any undesirable biological effect or by deleteriously interacting with any other component of the pharmaceutically acceptable composition, its use is contemplated as falling within the scope of the present invention. Some examples of materials which can serve as pharmaceutically acceptable carriers include, but are not limited to, ion exchangers, alumina, aluminum stearate, lecithin, serum proteins such as human serum albumin, Buffer substances such as phosphates, glycine, sorbic acid or potassium sorbate, mixtures of partial glycerides of saturated vegetable fatty acids, water, salts or electrolytes such as protamine sulphate, Disodium phosphate, potassium hydrogen phosphate, sodium chloride, zinc salts, colloidal silica, magnesium trisilicate, polyvinylpyrrolidone, polyacrylates, waxes, polyethylene-polyoxypropylene polymers, sugars such as lactose , Glucose and sucrose; Starches such as corn and potato starch; Cellulose and derivatives thereof such as sodium carboxymethylcellulose, ethylcellulose and cellulose acetate; Tragacanth powder; Malt; Gelatin; Talc; Excipients such as cocoa butter and suppository waxes; Oils such as peanut oil, cottonseed oil; Safflower oil; Sesame oil ; olive oil ; Corn oil and soybean oil; Glycols; Such a propylene glycol or polyethylene glycol; Esters such as ethyl oleate and ethyl laurate; Agar; Agents such as magnesium hydroxide and buffered aluminum hydroxide; Alginic acid; Isotonic saline; Ringer's solution; Ethyl alcohol, and phosphate buffer solutions, as well as other compatible non-toxic lubricants such as sodium lauryl sulfate and magnesium stearate, as well as coloring agents, release agents, coating agents, sweetening, flavoring and perfuming agents, preservatives and antioxidants may also be present in the composition, according to the judgment of the galenist.

The pharmaceutical form or method of administering a pharmaceutical composition may be selected with regard to the human or animal subject to be treated. For example it may be administered to humans and other animals orally, rectally, parenterally, intratracheally, intracisternally, intravaginally, intraperitoneally, topically (as by powders, ointments or drops), buccally, as oral or nasal spray, subcutaneously, or the like, depending on the severity of the infection to be treated. The pharmaceutical form or method of administering a pharmaceutical composition may be selected with regard to the site of infection and/or infected organ. For example, for an infection of the respiratory tract it may in a form adapted to be administered to humans and other animals as oral or nasal spray or parenteral or intratracheal, for an infection of the gastrointestinal tract it may in a form adapted to be administered to humans and other animals orally, for example a pellet, a capsule, a powder, granules, a syrup or parenteral or intraperitoneal. The pharmaceutical form or method of administering a pharmaceutical composition may be selected with regard to the age of the human to be treated, and/or with regard to comorbidity, associated therapies and/or site of infection. For example, for a child, for example from 1 to 17 years old, or a baby, for example under 1 year old, a syrup or an injection, for example subcutaneous or intravenous may be preferred. Administration may for example be carried out with a weight graduated pipette, a syringe. For example, for an adult over 17 years old, an injection may be preferred. Administration may be carried out with an intravenous weight graduated syringe.

According to the present invention, the pharmaceutical composition may comprise any pharmaceutically acceptable and effective amount of inhibitor of surface protein D (SP-D).

According to the present invention, the pharmaceutical composition may comprise any pharmaceutically acceptable and effective amount of inhibitor of surface protein D (SP-D)-SIRPα interaction.

According to the present invention, the pharmaceutical composition may comprise any pharmaceutically acceptable and effective amount of inhibitor of SHP-2
In this document, an "effective amount" of a pharmaceutically acceptable compound or composition according to the invention refers to an amount effective to treat or reduce the severity of nosocomial disease. The compounds and compositions for use according to the present invention may be administered using any amount and any route of administration effective to treat or reduce the severity of a nosocomial disease or condition associated with. The exact amount required will vary from one subject to another, depending on the species, age and general condition of the subject, the severity of the infection, the particular compound and its mode of administration.

Inhibitor of SP-D or inhibitor of SP-D/SIRPα interaction or inhibitor of SHP-2 for use according to the invention are preferably formulated in unit dosage form to facilitate dosing administration and uniformity. In this document, the term "unit dosage form" refers to a physically distinct unit of compound suitable for the patient to be treated. However, it will be understood that the total daily dosage of the compounds and compositions according to the present invention will be decided by the attending physician. The specific effective dose level for a particular animal or human patient or subject will depend on a variety of factors including the disorder or disease being treated and the severity of the disorder or disease; The activity of the specific compound employed; The specific composition employed; Age, body weight, general health, sex and diet of the patient / subject; The period of administration, the route of administration and the rate of elimination of the specific compound employed; duration of treatment; The drugs used in combination or incidentally with the specific compound used and analogous factors well known in the medical arts. The term "patient" as used herein refers to an animal, preferably a mammal, and preferably a human.

According to the present invention, the pharmaceutical composition may comprise effective amount of Inhibitor of SP-D and/or of SIRPA and/or SHP-2. For example, the pharmaceutical composition may comprise doses of inhibitor of SP-D and/or of SIRPA and/or SHP-2 adapted with regards to the nosocomial disease to be treated and/or to the subject to be treated. One skilled in the art taking into consideration his technical knowledge would adapt the amount in the pharmaceutical composition with regard to the nosocomial disease to be treated and/or to the subject to be treated. For example the pharmaceutical composition may comprise inhibitor of SP-D and/or of SIRPA and/or SHP-2 at doses about 0.01 to 2000 µg, for example about 0.01 to 1000 µg, preferably from 1 to 100 µg. For example, the pharmaceutical composition may comprise Inhibitor of SP-D in an amount allowing administration of Inhibitor of SP-D at doses of from about 0.1 µg/kg to 1 µg/Kg body weight of the subject.

According to the invention, inhibitor of SP-D and/or of SIRPA and/or SHP-2 may be administered on a single administration or repeated administrations, for example one to three time per day.

According to the invention, Inhibitor of SP-D and/or of SIRPA and/or SHP-2 may be administered for example for a period from 1 to 90 days, for example from 1 to 7 days.

According to the present invention, the pharmaceutical composition may comprise any pharmaceutically acceptable and effective amount of inhibitor of surface protein D (SP-D)-SIRPα interaction. For example, the pharmaceutical composition may comprise doses of inhibitor of surface protein D (SP-D)-SIRPα adapted with regards to the inhibitor used. One skilled in the art taking into consideration his technical knowledge would adapt the amount in the pharmaceutical composition with regard to the used inhibitor. For example, when the inhibitor of surface protein D (SP-D)-SIRPα interaction is a small molecules, for example NSC-87877, the pharmaceutical composition may comprise, for example at doses around 10 mg. For example, when the inhibitors of surface protein D (SP-D)- SIRPα interaction is humanized antibody, for example P84, the pharmaceutical composition may comprise, for example at doses from 0.1 to 100 mg.

According to the invention, inhibitors of surface protein D (SP-D)-SIRPα interaction may be administered on a single time or repeated administration, for example one to three time per day.

According to the invention, inhibitors of surface protein D (SP-D)-SIRPα interaction may be administered for example for a period from 1 to 90 days, for example from 1 to 7 days.

According to the present invention, the pharmaceutical composition may comprise any pharmaceutically acceptable and effective amount of inhibitor of SHP2. For example, the pharmaceutical composition may comprise doses of inhibitor of inhibitor of SHP2 adapted with regards to the inhibitor used. One skilled in the art taking into consideration his technical knowledge would adapt the amount in the pharmaceutical composition with regard to the used inhibitor. For example, when the inhibitor of SHP2 is a small molecules, for example for NSC-87877 or SHP099, the pharmaceutical composition may comprise, for example at doses around 10 mg. For example, when the inhibitors surface protein D (SP-D)- SIRPα interaction is humanized antibody, for example P84, the pharmaceutical composition may comprise, for example at doses from 0.1 to 100 mg.

According to the invention, inhibitors of SHP2 may be administered on a single time or repeated administration, for example one to three time per day.

According to the invention, inhibitors of SHP2 may be administered for example for a period from 1 to 90 days, for example from 1 to 7 days.

According to another aspect, the present disclosure relates to an inhibitor of SP-D, or pharmaceutical composition comprising inhibitor of SP-D, for its use as a medicament, in particular in the treatment of secondary infection.

The inhibitor of SP-D is as defined above.

The pharmaceutical composition comprising inhibitor of SP-D is as defined above.

The secondary infection is as defined above. For example secondary infection may be nosocomial diseases, it may be for example pneumonia, pleural infection, urinary infection, peritoneal infection, intra-abdominal abscess, meningitis, mediastinal infection and soft-tissue or skin infection

According to another aspect, the present disclosure relates to an inhibitor of SP-D/SIRPα interaction, or pharmaceutical composition comprising inhibitor of SP-D/SIRPα interaction, for its use as a medicament, in particular in the treatment of secondary infection.

The inhibitor of SP-D/SIRPα interaction is as defined above.

The pharmaceutical composition comprising inhibitor of SP-D/SIRPα is as defined above.

The secondary infection is as defined above. For example secondary infection may be nosocomial diseases, it may be for example pneumonia, pleural infection, urinary infection, peritoneal infection, intra-abdominal abscess, meningitis, mediastinal infection and soft-tissue or skin infection

According to another aspect, the present disclosure relates to an inhibitor of SPH-2, or pharmaceutical composition comprising inhibitor of SPH2, for its use as a medicament, in particular in the treatment of secondary infection.

The inhibitor of SPH-2 is as defined above.

The pharmaceutical composition comprising inhibitor of SPH-2is as defined above.

According to another aspect, the present disclosure relates to an inhibitor of SP-D and/or an inhibitor of SP-D/SIRPα interaction and/or an inhibitor of SPH-2, or pharmaceutical composition comprising inhibitor of SPH2, for its use as a medicament, in particular in the treatment of secondary infection.

The inhibitor of SPH-2 is as defined above.

The pharmaceutical composition comprising inhibitor of SPH-2is as defined above.

The secondary infection is as defined above. For example secondary infection may be nosocomial diseases, it may be for example pneumonia, pleural infection, urinary infection, peritoneal infection, intra-abdominal abscess, meningitis, mediastinal infection and soft-tissue or skin infection

According to another aspect, the present disclosure relates to a method of treating or preventing secondary diseases comprising administering an effective amount of inhibitor of SP-D or composition comprising inhibitor of SP-D to a subject.

According to another aspect, the present disclosure relates to a method of treating or preventing secondary diseases comprising administering an effective amount of inhibitor of SP-D/SIRPα interaction or composition comprising inhibitor of SP-D/SIRPα interaction to a subject.

According to another aspect, the present disclosure relates to a method of treating or preventing secondary diseases comprising administering an effective amount of inhibitor of SPH2 or composition comprising inhibitor of SPH2 a subject.

The inhibitor of SP-D is as defined above.

The inhibitor of SP-D/SIRPα interaction is as defined above.

The inhibitor of SPH2 is as defined above.

The composition comprising inhibitor of SP-D is as defined above.

The composition comprising inhibitor of SP-D/SIRPα interaction is as defined above.

The composition comprising inhibitor of SPH2 is as defined above.

The secondary infection is as defined above. For example, secondary infection may be nosocomial diseases, it may be for example pneumonia, pleural infection, urinary infection, peritoneal infection, intra-abdominal abscess, meningitis, mediastinal infection and soft-tissue or skin infection.

The administration of inhibitor of SP-D and/or SP-D/SIRPα interaction and/or SPH2 interaction or composition comprising inhibitor of SP-D and/or SP-D/SIRPα interaction and/or SPH2 may be carried out by any way/routes known to the skilled person. For example it may be administered in any form and/or way/routes as mentioned above.

According to another aspect, the present disclosure relates to a method of treating or preventing secondary diseases comprising administering an effective amount of inhibitor of SP-D and/or SP-D/SIRPα interaction and/or SPH2.

The inhibitor of SP-D is as defined above.

The inhibitor of SP-D/SIRPα interaction and/or SPH2 is as defined above.

The secondary infection is as defined above. For example secondary infection may be nosocomial diseases, it may be for example pneumonia, pleural infection, urinary infection, peritoneal infection, intra-abdominal abscess, meningitis, mediastinal infection and soft-tissue or skin infection.

The administration of composition comprising inhibitor of SP-D and/or SP-D/SIRPα interaction and/or SPH2 may be carried out by any way/routes known to the skilled person. For example it may be administered in any form and/or way/routes as mentioned above.

The medicament may be in any form that can be administered to a human or an animal. It may for example be a pharmaceutical composition as defined above.

The administration of the medicament may be carried out by any way known to one skilled in the art. It may, for example, be carried out directly, i.e. pure or substantially pure, or after mixing of the antibody or antigen-binding portion thereof with a pharmaceutically acceptable carrier and/or medium. According to the present invention, the medicament may be an injectable solution, a medicament for oral administration, for example selected from the group comprising a liquid formulation, a multiparticle system, an orodispersible dosage form. According to the present invention, the medicament may be a medicament for oral administration selected from the group comprising a liquid formulation, an oral effervescent dosage form, an oral powder, a multiparticle system, an orodispersible dosage form.

The inhibitor of SP-D and/or SP-D / SIRPα interaction and/or SPH2 as described above and pharmaceutically acceptable compositions for use according to the present invention may also be used in combination therapies, i.e., compounds and pharmaceutically acceptable compositions may be administered simultaneously with, before or after one or more other therapeutic agents, or medical procedures. The particular combination of therapies (therapies or procedures) to be employed in an association scheme will take into account the compatibility of the desired therapeutic products and / or procedures and the desired therapeutic effect to be achieved. The therapies used may be directed to the same disease (for example, a compound according to the invention may be administered simultaneously with another agent used to treat the same disease), or may have different therapeutic effects (eg, undesirable).

For example, therapeutic agents known to treat secondary disease, for example nosocomial diseases, for example antibiotics, antifungal and/or antiviral compounds and/or antibacterial antibody and/or interferon therapy. It may be for example any antibiotic known to one skilled in the art. It may be for example antibiotic used for the treatment of pneumonia, pleural infection, urinary infection, peritoneal infection, intra-abdominal abscess, meningitis, mediastinal infection. It may be for example antibiotic selected from the group comprising Gentamicin, Kanamycin, Neomycin, Netilmicin, Tobramycin, Paromomycin, Streptomycin, Spectinomycin, Geldanamycin, Herbimycin, Rifaximin, Loracarbef, Ertapenem, Doripenem, Imipenem/Cilastatin, Meropenem, Cefadroxil, Cefazolin, Cefalotin or Cefalothin, Cefalexin, Cefaclor, Cefamandole, Cefoxitin, Cefprozil, Cefuroxime, Cefixime; Cefdinir; Cefditoren, Cefoperazone, Cefotaxime, Cefpodoxime, Ceftazidime, Ceftibuten, Ceftizoxime, Ceftriaxone, Cefepime, Ceftaroline fosamil, Ceftobiprole, Ceftolozane, Avibactam, Teicoplanin, Vancomycin, Telavancin, Dalbavancin, Oritavancin, Clindamycin, Lincomycin, Daptomycin, Azithromycin, Clarithromycin, Dirithromycin, Erythromycin, Roxithromycin, Troleandomycin, Telithromycin, Spiramycin, Aztreonam, Furazolidone, Nitrofurantoin, Linezolid, Tedizolid, Posizolid, Radezolid, Torezolid, Amoxicillin, Ampicillin, Azlocillin, Carbenicillin, Cloxacillin, Dicloxacillin, Flucloxacillin, Mezlocillin, Methicillin, Nafcillin, Oxacillin, Penicillin G, Penicillin V, Piperacillin, Temocillin, Ticarcillin, Amoxicillin/clavulanate, Ampicillin/sulbactam, Piperacillin/tazobactam, Ticarcillin/clavulanate, Bacitracin, Colistin, Polymyxin B, Ciprofloxacin, Enoxacin, Gatifloxacin, Gemifloxacin, Levofloxacin, Lomefloxacin, Moxifloxacin, Nalidixic acid, Norfloxacin, Ofloxacin, Trovafloxacin, Grepafloxacin, Sparfloxacin, Temafloxacin, Mafenide, Sulfacetamide, Sulfadiazine, Silver sulfadiazine, Sulfadimethoxine, Sulfamethizole, Sulfamethoxazole, Sulfanilimide, Sulfasalazine, Sulfisoxazole, Trimethoprim-Sulfamethoxazole, Sulfonamidochrysoidine, Demeclocycline, Doxycycline, Minocycline, Oxytetracycline, Tetracycline, Clofazimine, Dapsone, Capreomycin, Cycloserine, Ethambutol(Bs), Ethionamide, Isoniazid, Pyrazinamide, Rifampicin, Rifabutin, Rifapentine, Streptomycin, Arsphenamine, Chloramphenicol(Bs), Fosfomycin, Fusidic acid, Metronidazole, Mupirocin, Platensimycin, Quinupristin/Dalfopristin, Thiamphenicol, Tigecycline(Bs), Tinidazole, Trimethoprim(Bs).

It may be for example antifungal compound selected from the group comprising Bifonazole, Butoconazole, Clotrimazole, Econazole, Fenticonazole, Isoconazole, Ketoconazole, Luliconazole, Miconazole, Omoconazole, Oxiconazole, Sertaconazole, Sulconazole, Tioconazole, Amphotericin B, Candicidin, Filipin, Hamycin, Natamycin, Nystatin, Rimocidin, Albaconazole, Efinaconazole, Epoxiconazole, Fluconazole, Isavuconazole, Itraconazole, Posaconazole, Propiconazole, Ravuconazole, Terconazole, Voriconazole, Abafungin, Anidulafungin, Caspofungin, Micafungin, Aurones, Benzoic acid, Ciclopirox, Flucytosine or 5-fluorocytosine, Griseofulvin, Haloprogin, Tolnaftate, Undecylenic acid.

It may be for example antiviral compound selected from the group comprising Abacavir, Acyclovir, Adefovir, Amantadine, Amprenavir, Ampligen, Arbidol, Atazanavir, Atripla, Balavir, Cidofovir, Combivir, Dolutegravir, Darunavir, Delavirdine, Didanosine, Docosanol, Edoxudine, Efavirenz, Emtricitabine, Enfuvirtide, Entecavir, Ecoliever, Famciclovir, Fomivirsen, Fosamprenavir, Foscarnet, Fosfonet, Fusion inhibitor, Ganciclovir, Ibacitabine, Imunovir, Idoxuridine, Imiquimod, Indinavir, Inosine, Integrase inhibitor, Interferon type III, Interferon type II, Interferon type I, Interferon, Lamivudine, Lopinavir, Loviride, Maraviroc Moroxydine, Methisazone, Nelfinavir, Nevirapine, Nexavir, Nitazoxanide, Nucleoside analogues, Novir, Oseltamivir, Peginterferon alfa-2a, Penciclovir, Peramivir, Pleconaril, Podophyllotoxin, Protease inhibitor, Raltegravir, Reverse transcriptase inhibitor, Ribavirin, Rimantadine, Ritonavir, Pyramidine, Saquinavir, Sofosbuvir, Stavudine, Telaprevir, Tenofovir, Tenofovir disoproxil, Tipranavir, Trifluridine, Trizivir, Tromantadine, Truvada, Valaciclovir, Valganciclovir, Vicriviroc, Vidarabine, Viramidine, Zalcitabine, Zanamivir, Zidovudine.

The inventors have surprisingly demonstrated that the concentration of SP-D could be considered as a biomarker of the capacity of the immune defense to protect and/or to respond to a pathogen subsequently encountered. In other words, the inventors have surprisingly demonstrated that the concentration of SP-D is a marker of the susceptibility of a subject to an infection, in particular to a secondary infection.

Accordingly, another object of the invention is the use of surface protein D (SP-D) as a biomarker of a secondary infection.

According to the invention, the surface protein D (SP-D) may be used in any process and/or method as a biomarker of a secondary infection.

The inventors have also demonstrated that the concentration of SP-D is increased in subject susceptible to secondary disease and/or nosocomial disease. In particular, the inventors have demonstrated that the concentration of SP-D is increased in subject with deficient or less reactive immune response to a pathogen.

Another object of the present invention is an ex *vivo* method for determining the susceptibility to a secondary disease of a subject comprising
a. Determining the concentration(Cₛ₁) and /or expression level (Lₛ₁) of surface protein D (SP-D) in a biological sample of said subject,
b. Comparison of the measured concentration Cₛ₁ and /or expression Lₛ₁ with a corresponding reference value Cₛᵣₑf and /or L_{sref} by calculating a score S3= Cₛ₁/C_{ref} and/or S4= Lₛ₁/L_{sref},
c. Determining the susceptibility to a secondary disease according to the value of score S3 and/or S4 obtained:
   - If S3 > 1 and/or S4 > 1 , the subject is considered likely to have a deficiency in immunity response to any pathogen, or,
   - If S3 ≤ 1 and/or S4 ≤ 1, the subject is considered unlikely to have a deficiency in immunity response to any pathogen.

The term "subject" as used herein refers to an animal, preferably a mammal, and preferably a human.

In the present, "deficiency in immunity" means that the subject may have decreased immunogenic response and/or capacity of initiating adaptive and/or capacity of activating innate immunity with regards to a pathogen and/or a reduction of the activation or efficacy of the immune system.

In the present, "susceptibility to a secondary disease" means a subject having a reduction of the activation or efficacy of the immune system and/or having an increased susceptibility to opportunistic infections and decreased cancer immunosurveillance.

According to the invention, "biological sample" means a liquid or solid sample. According to the invention, the sample can be any biological fluid, for example it can be a sample of blood, of plasma, of serum, of cerebrospinal fluid, of respiratory fluid, of vaginal mucus, of nasal mucus, of saliva and/or of urine. Preferably the biological sample is a respiratory fluid, for example selected from the group consisting of a sample of tracheal fluid, bronchoalveolar lavages, and/or pleural fluid.

According to the invention the concentration level of surface protein D (SP-D) may be determined by any method or process known from one skilled in the art. It may be for example determined with immunoassay, for example with ELISA and/or radioimmunoassay.

According to the invention the concentration level of surface protein D (SP-D) may be determined from any biological sample. For example, the concentration of surface protein D (SP-D) may be determined respiratory fluid, for example selected from the group consisting of a sample of tracheal fluid, bronchoalveolar lavages, and/or pleural fluid. It may be preferably determined from tracheal fluid.

According to the invention, the referenced level of concentration of surface protein D (SP-D) (C_{ref} or C_{sref}) may be the mean concentration level of surface protein D (SP-D) (C_{ref} or C_{sref}) in subject without any disease and/or which has not been infected with a pathogen at least since two weeks. For example the referenced level of concentration of surface protein D (SP-D) may be between 1 to 1000 mg/mL. For example the referenced level of concentration of surface protein D (SP-D) may be between 1 pg/mL to 1000 mg/mL. For example the reference value C_{sref} may be from 1 pg/mL to 1000 mg/mL
According to the invention the expression of surface protein D (SP-D) may be determined by any method or process known from one skilled in the art. It may be for example determined with flow cytometry, RT-PCR or any method disclosed in Marcel Geertz and Sebastian J. Maerkl, Experimental strategies for studying transcription factor-DNA binding specificities, Brief Funct Genomics. 2010 Dec; 9(5-6): 362-373 [28].

The inventors have also advantageously demonstrate that the invention makes it possible to establish, before any secondary disease and/or nosocomial disease whether a subject may be more susceptible to such disease and whether the condition of a such can be improved by administration of a treatment, in particular a treatment improving and/or restoring the immunity response as the medicament of the invention i.e. SP-D inhibitor and/or inhibitor of SP-D / SIRPα interaction.

The inventors have also demonstrated that the expression and concentration of SIRPα is decreased in subject susceptible to secondary disease and/or nosocomial disease. In particular, the inventors have demonstrated that the concentration of SIRPα is involved in the maintenance, in subject, of deficient or less reactive immune response to a pathogen. In other words, the inventors have demonstrated that the expression and/or concentration of SIRPα may be correlated with a secondary infection.

Accordingly, another object of the invention is the use of surface protein D (SP-D) as a biomarker of a secondary infection.

According to the invention, the surface protein D (SP-D) may be used in any process and/or method as a biomarker of a secondary infection.

Another object of the present invention is an ex *vivo* method for determining the susceptibility to a secondary disease of a subject comprising
a. Determining the concentration(Cₐ₁) and /or expression level (Lₐ₁) of SIRPα in a biological sample of said subject,
b. Comparison of the measured concentration Cₐ₁ and /or expression Lₐ₁ with a corresponding reference value C_{aref} and /or L_{aref} by calculating a score S5= Cₐ₁/ C_{aref} and/or S6= Lₐ₁/L_{aref},
c. Determining the susceptibility to a secondary disease of a subject according to the value of score S5 and/or S6 obtained:
   - If S5 > 1 and/or S6 > 1 , the subject is considered likely to have a deficiency in immunity response to any pathogen, or,
   - If S5 ≤ 1 and/or S6 ≤ 1, the subject is considered unlikely to have a deficiency in immunity response to any pathogen.

The "subject" is as mentioned above

The "biological sample" is as mentioned above, preferably the biological sample is a respiratory fluid, for example selected from the group consisting of a sample of tracheal fluid, bronchoalveolar lavages, and/or pleural fluid.

According to the invention the concentration level of SIRPα may be determined by any method or process known from one skilled in the art. It may be for example determined with immunoassay, for example with ELISA and/or radioimmunoassay.

According to the invention the concentration level of SIRPα may be determined from any biological sample. For example, the concentration of SIRPα may be determined from respiratory fluid, for example selected from the group consisting of a sample of tracheal fluid, bronchoalveolar lavages, pleural fluid and/or blood. It may be preferably determined from blood.

According to the invention the expression of SIRPα may be determined by any method or process known from one skilled in the art. It may be for example determined with flow cytometry, RT-PCR or any method disclosed in Marcel Geertz and Sebastian J. Maerkl, Experimental strategies for studying transcription factor-DNA binding specificities, Brief Funct Genomics. 2010 Dec; 9(5-6): 362-373 [28].

According to the invention the expression level of SIRPα may be determined from any immune cell of the biological sample. For example, the expression level of SIRPα may be determined from cell selected from the group myeloid cells. It may be preferably determined from circulating monocytes.

According to the invention, the referenced level of expression of SIRPα (L_{aref}) may be the mean expression level of SIRPα (L_{aref}) in subject without any disease and/or which has not been infected with a pathogen at least since two weeks. For example the referenced level of expression of SIRPα may be between 10000 to 20000 gMFI on monocytes. For example the reference value C_{aref} may be from 1 pg/mL to 100 mg/mL.

The inventors have also advantageously demonstrate that the invention makes it possible to establish, before any secondary disease and/or nosocomial disease whether a subject may be more susceptible to such disease and whether the condition of a such can be improved by administration of a treatment, in particular a treatment improving and/or restoring the immunity response as the medicament of the invention i.e. SP-D inhibitor and/or inhibitor of SP-D / SIRPα interaction and/or inhibitor of SHP-2.

Other advantages may still be apparent to those skilled in the art by reading the examples below, illustrated by the accompanying figures, given by way of illustration.

### Brief description of the Figures

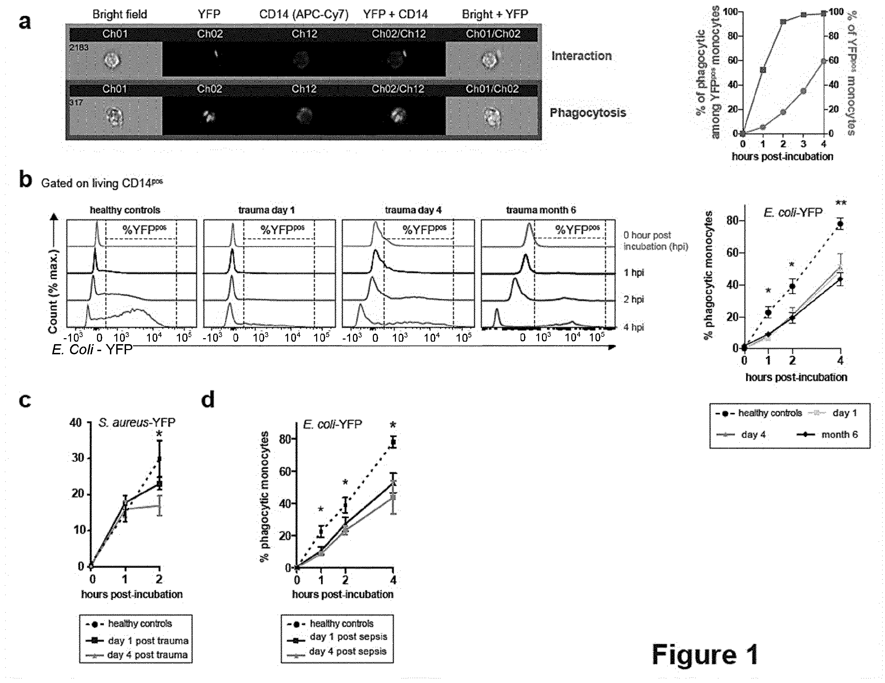

**Figure 1** represents the recovery from infection or trauma is followed by a reduction of phagocytosis of extracellular bacteria by monocytes in humans. Figure 1a comprises images of monocytes (CD14pos) and a histogram. The images were taken by imaging flow cytometry at the indicated time after incubation with YFP- Escherichia coli (E. coli) of peripheral blood mononuclear cells collected from healthy humans (n = 3). Representative of 2 independent experiments, the histogram present the percentage of phagocytic monocyte (ordinate) in light of the time, in hours post incubation (abscissa). Figure 1b is histograms, representative histograms and percentages of YFP levels in CD14pos cells after 0, 1, 2 or 4 hours post-incubation (hpi) with YFP-E. coli of PBMCs collected in healthy uninfected humans and from patients from day 1 to month 6 after severe trauma, (n = 5-7 patients). Graphs represent mean ± SD and are pooled data from 3 independent experiments. The histogram present the percentage of phagocytic monocyte (ordinate) in light of the time, in hours post incubation (abscissa). Figure 1 c is an histogram representing the percentages of phagocytic (YFPpos) monocytes (CD14pos) after incubation with YFP-Staphylococcus aureus (S. aureus) of PBMC collected from healthy uninfected humans or at the indicated time after severe trauma (n = 5-7 patients). Graphs represent mean ± SD and are pooled data from 3 independent experiments. Figure 1 d is an histogram representing the percentages of phagocytic (YFPpos) monocytes (CD14pos) after incubation with YFP-E. coli of PBMC collected from healthy uninfected humans or from severe septic patients at the indicated time after sepsis onset (n = 5-7 patients). Graphs represent mean ± SD and are pooled data from 3 independent experiments.

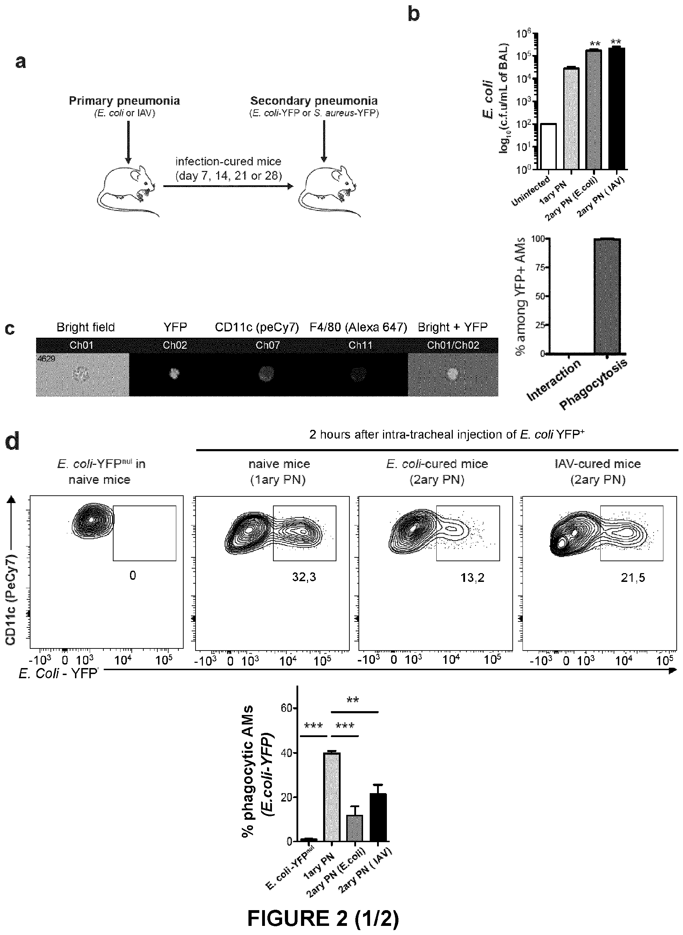

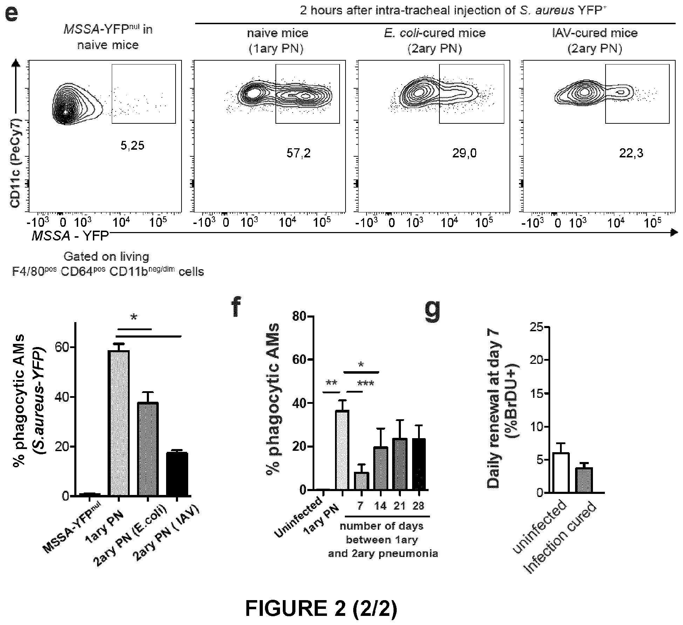

**Figure 2** shows that the recovery from infection is followed by susceptibility to Secondary Pneumonia and reduction in phagocytosis by alveolar macrophages in mice. Figure 2 a is a schematic diagram of the experimental outline of primary pneumonia with Escherichia coli (E. coli) or influenza A virus (IAV) and secondary pneumonia with YFPpos-E. coli or YFPpos-S. aureus. Figure 2 b is an histogram representing the enumeration of colony-forming units (CFU) per milliliter of bronchoalveolar lavage (CFU/mL) (ordinate) analyzed one day after secondary E. coli pneumonia induced 7 days after E. coli or IAV primary pneumonia (n = 5 mice per group) (abscissa). Figure 2 c is image of AM (F4/80posCD11cpos) from bronchoalveolar lavage taken by imaging flow cytometry 2 hours after intratracheal instillation of YFP-E. coli in mice (n = 3 mice). Representative of 1 experiment. The histogram represent the percentage of phagocytic cells and the percentage of unspecific binding. Figure 2d is diagrams comprising representative plots and percentages of phagocytic AM 2 hours after YFPneg or YFPpos-E. coli pneumonia (75µL, OD600=2-3) in naive mice (1ary pneumonia) or in mice cured from E. coli or IAV (influenza A virus) pneumonia (2ary pneumonia). (n=5-8 mice). Graphs represent mean ± SD and are pooled data from 3 independent experiments. The histogram represent the percentage of phagocytic alveolar macrophages. Figure 2e is diagrams comprising representative plots and percentages of phagocytic AM 2 hours after YFPneg or YFPpos-MSSA pneumonia (75µL, OD600=2-3) in naive mice (1 ary pneumonia) or in mice cured from E. coli or IAV pneumonia (2ary pneumonia). (n=4-5 mice). Graphs represent mean ± SD and are pooled data from 2 independent experiments. The histogram represent the percentage of phagocytotic AM (ordinate) with regards in naive mice (1ary pneumonia) or in mice cured from E. coli or IAV pneumonia (2ary pneumonia). Figure 2 f is a histogram representing the percentages of phagocytic AM (ordinate) 2 hours after YFP-E. coli pneumonia during secondary pneumonia realized at the indicated time point after E. coli 1ary pneumonia (n >10 mice at day 7, n = 3-4 at day 14, 21, and 28 (abscissa)). Graphs represent mean ± SD from 1 experiment. Figure 2 g is an histogram representing the daily renewal at day 7 (ordinate) after intraperitoneal injection of BrDU (1 mg per day for 2 days) in uninfected mice, and in infection-cured mice (5-7 days after E. coli injection). Percentage of BrDU+ alveolar macrophages was assessed by flow cytometry (n=3 mice per group). Graphs represent mean ± SD and are pooled data from 2 independent experiments. *p<0.05, **p<0.01, ***p<0.001

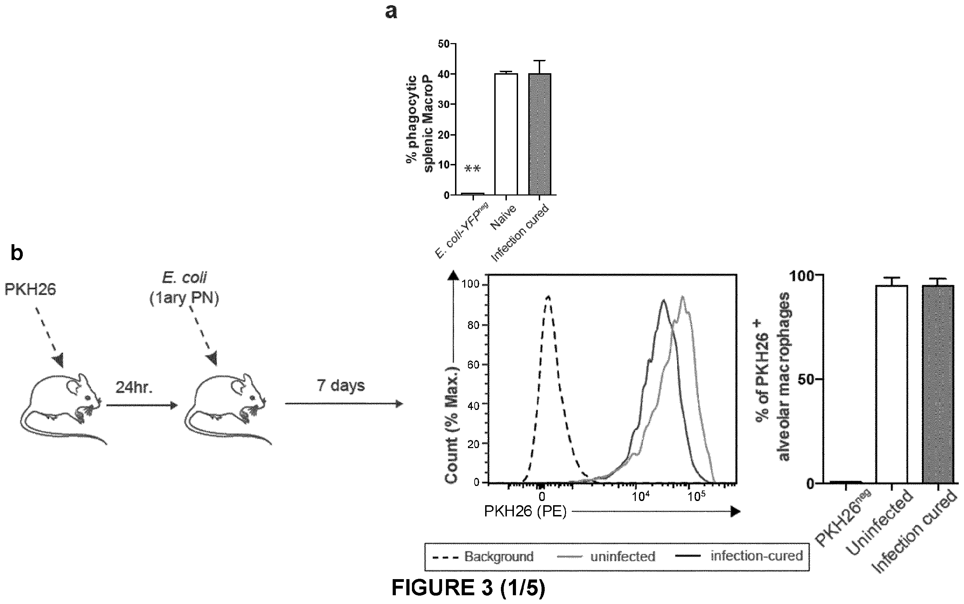

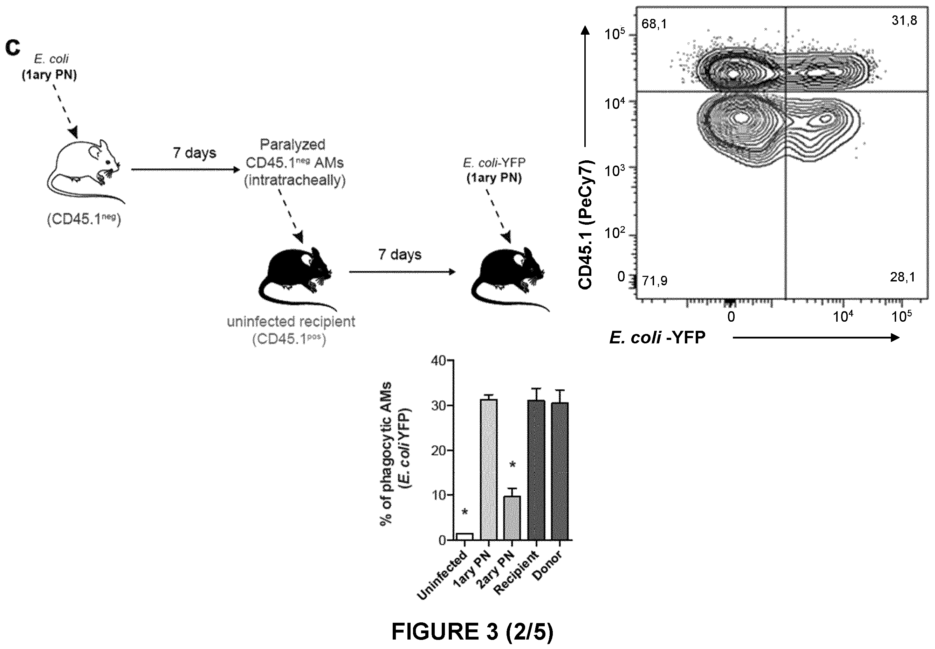

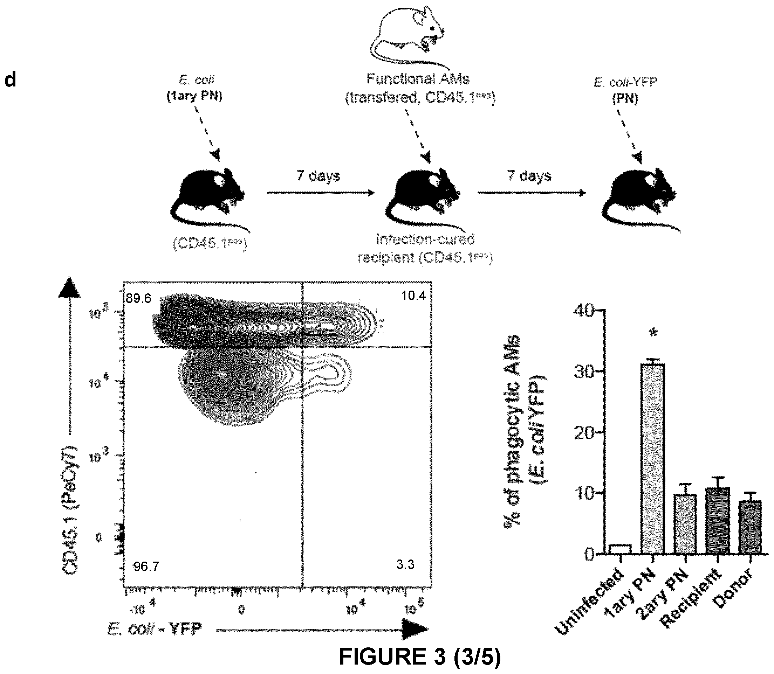

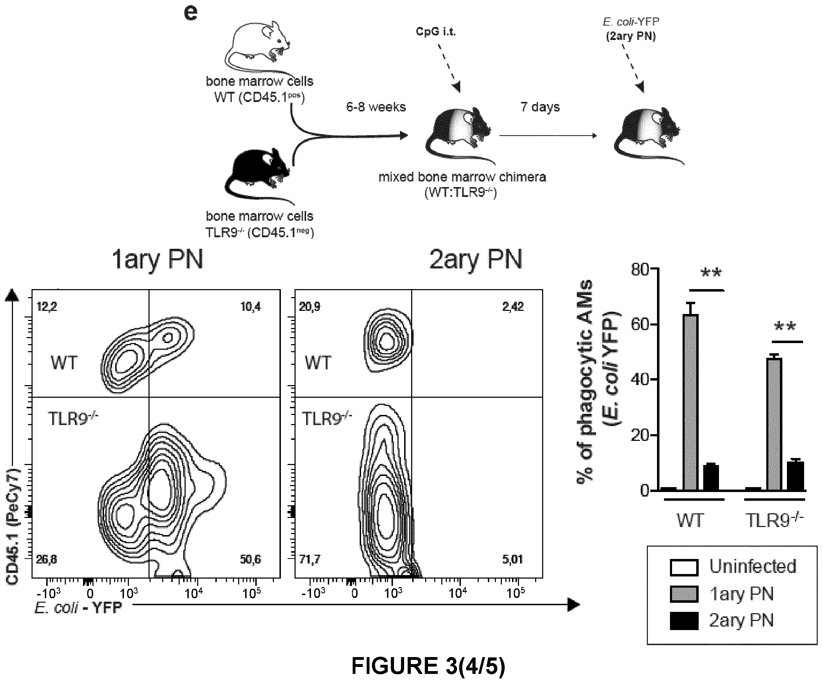

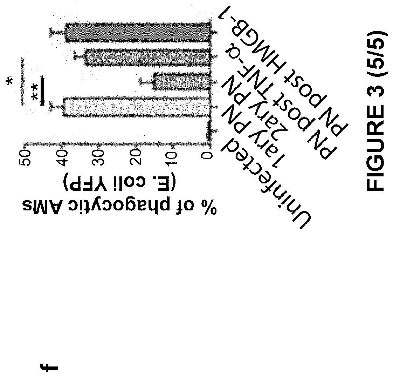

**Figure 3** demonstrates that phagocytosis function of newly formed resident lung alveolar macrophages (AM) is altered locally by secondary inflammatory mediators released during infection. Figure 3a is a histogram representing the frequencies of phagocytic splenic macrophages (ordinate) were measured 2 hpi with YFPpos-E. coli in naive mice or E. coli pneumonia-cured (abscissa). (n=4 mice per group). Graphs represent mean± SD and are pooled data from 2 independent experiments. Figure 3b represent a diagram showing the proportions of resident alveolar macrophages (PKH26pos cells) (abscissa), a schema representing mice were intratracheally injected with PKH26-phagocytic cell linker 24 hours before the induction of E. coli pneumonia. 7 days later, the proportions of resident alveolar macrophages (PKH26pos cells) were measured in uninfected and in infection-cured mice. (n=5 mice per group). The histogram represents the percentage of alveolar macrophages (PKH26pos cells) (ordinate) with regards to uninfected or infection cured mice (abscissa) Graphs represent mean ± SD and are pooled data from 2 independent experiments. Figure 3 c represents a schema of the protocol of infection: recipient mice (CD45.1 pos) were intra-tracheally injected with AM collected from infection-cured donors (CD45.1neg). 7 days later, recipients were injected with YFP-E. coli (i.t.) and the percentages of phagocytic cells were assessed in donor and recipient AM (n = 4 mice per group). The diagram represent the number of cells with regards to the paralyzed or uninfected mice. The histogram represent that percentage of phagocytic AM (ordinate) with regards to uninfected, primary infected, secondary infected, the recipient or donor mice (abscissa) Graphs represent mean ± SD from 1 experiment. Figure 3d represents a schema of the protocol of infection: recipient mice (CD45.1pos) were infected with E. coli and 7 days later they were intra-tracheally injected with AM collected from uninfected donors (CD45.1neg). 7 days later, recipients were injected with YFP-E. coli (i.t.) and the percentages of phagocytic cells were assessed on donor and recipient AM (n = 4 mice per group). The diagram represent the number of cells with regards to the paralyzed or uninfected mice. The histogram represent that percentage of phagocytic AM (ordinate) with regards to uninfected, primary infected, secondary infected, the recipient or donor mice (abscissa) Graphs represent mean ± SD and are pooled data from 2 independent experiments. Figure 3 e represents a schema of the protocol of infection: lethally irradiated WT (CD45.1pos) recipient mice were reconstituted with 1:1 WT(CD45.1pos) and TLR9-/-(CD45.1neg) (which produces TRL9 deficient AM unable to respond to CpG). Frequencies of phagocytic lung AM were measured after induction of YFP+-E. coli pneumonia in (WT:TLR9-/-) mixed bone marrow chimeras intratracheally inoculated (so-called 2ary PN) or not (so-called 1ary PN) with CpG 7 days prior (n=3 mice per group). The diagram represent the number of cells with regards to the primary infected or secondary infected. The histogram represent that percentage of phagocytic AM (ordinate) with regards to uninfected, primary infected, secondary infected, the recipient or donor mice (abscissa) Graphs represent mean ± SD and are pooled data from 2 independent experiments. Figure 3 f is a histogram representing the percentages of phagocytic AM (ordinate) 2 hours after YFPpos-E. coli pneumonia (75µL, OD600=2-3) in naive mice (1ary PN) or in mice cured from E. coli pneumonia (2ary PN), or in mice intratracheally instilled with TNF-α (PN post TNF- α) or HMGB1 (PN post HMGB1) 7 days prior (abscissa). (n=4 mice per group). Graphs represent mean ± SD from 2 independent experiments. *p<0.05, **p<0.01, ***p<0.001

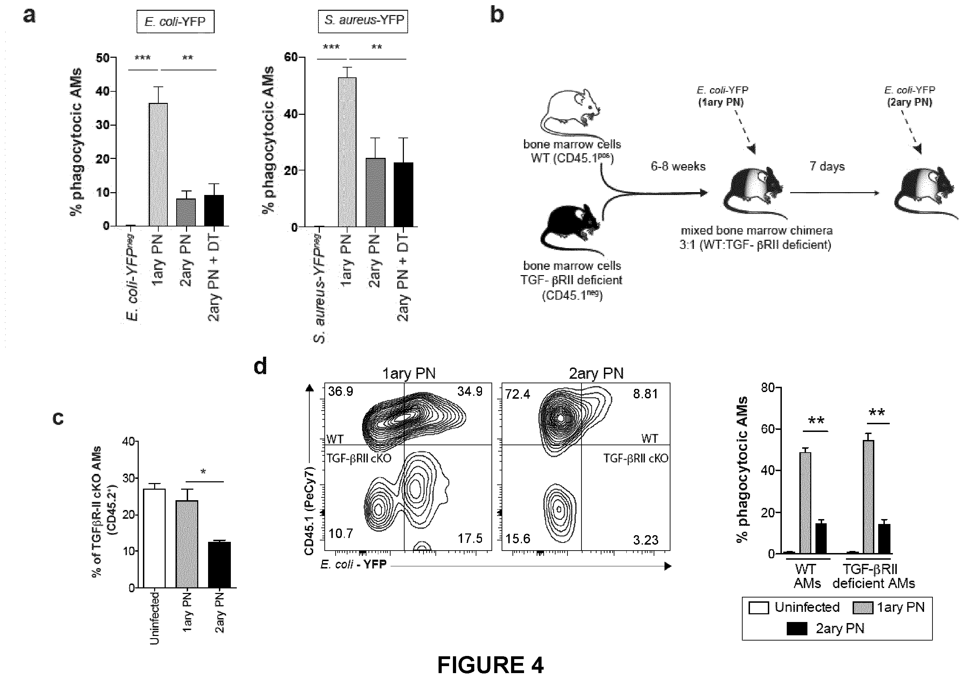

**Figure 4** demonstrates Treg cells and TGFβ are not major contributors to the paralysis program of alveolar macrophages. Figure 4a are histogram representing the percentage of phagocytic AMs (ordinate) with regards to the non infected mice, primary infected, secondary infected and secondary infected treated with diphtheria toxin (abscissa). The figure represent the frequencies of phagocytic AM were measured after induction of secondary YFP-E. coli or YFP-S. aureus pneumonia in FoxP3-DTR mice (DEREG) treated or not with diphtheria toxin (DT, 0.2 µg ip. day+3 and day+6 after 1ary pneumonia) (n=5 mice per group). Graphs represent mean ± SD and are pooled data from 2 independent experiments. Figure 4 b represents a schema of the protocol: Lethally irradiated WT CD45.1+ recipient mice were reconstituted with a 3:1 ratio of CD45.1+ WT and CD45.2+ TGF-βRIIfl/fl CD11 cCRE (which produces TGF-βRII-deficient AM). Figure 4 c is an histogram representing eight weeks after immune reconstitution, the percentages of CD45.1neg TGF-βRII-deficient AM (ordinate) were measured in uninfected chimeras, during primary or secondary pneumonia (abscissa) (n=4 mice per group). Graphs represent mean ± SD and are pooled data from 2 independent experiments. Figure 4 d is a diagram representing the frequencies of phagocytic AM measured after induction of primary or secondary YFP-E. coli pneumonia in (WT:TGF-βRII deficient AM) mixed bone marrow chimeras (3:1 ratio) (n=4 mice per group), the histogram represent the percentage of phagocytic AM (ordinate) with regards to the uninfected, primary infected and secondary infected mice comprising WT or TGF-βRII deficient AM Graphs represent mean ± SD and are pooled data from 2 independent experiments. *p<0.05, **p<0.01, ***p<0.001

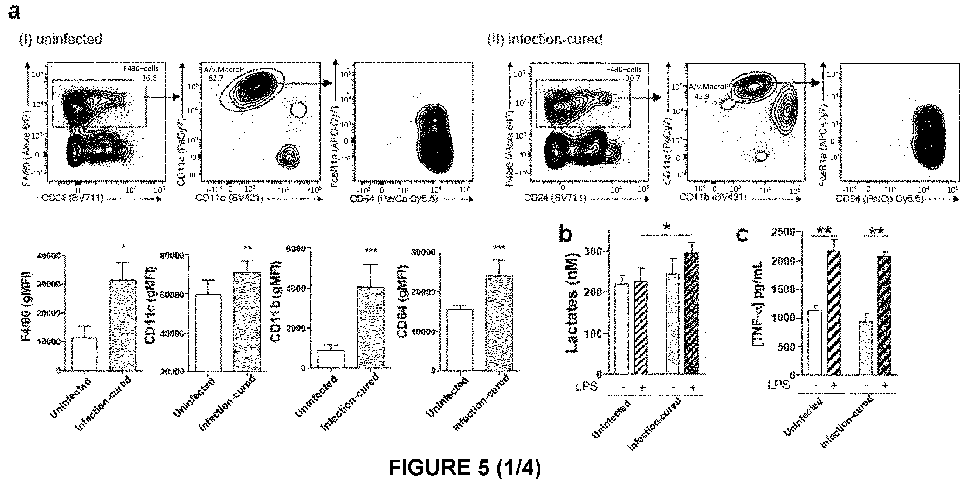

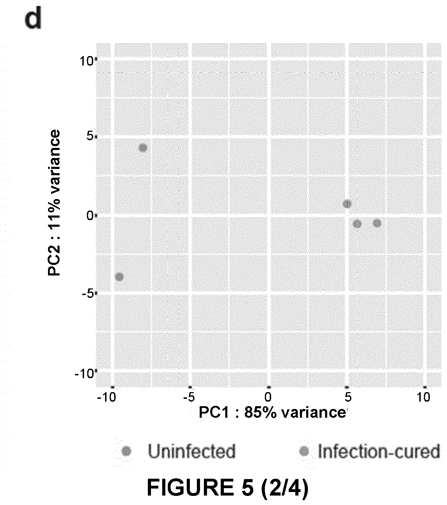

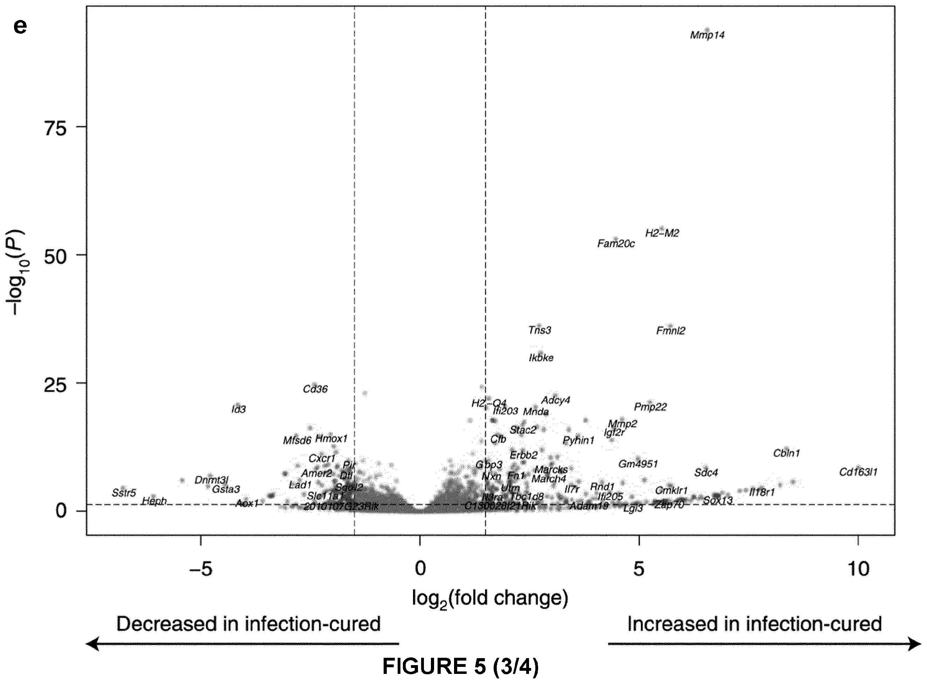

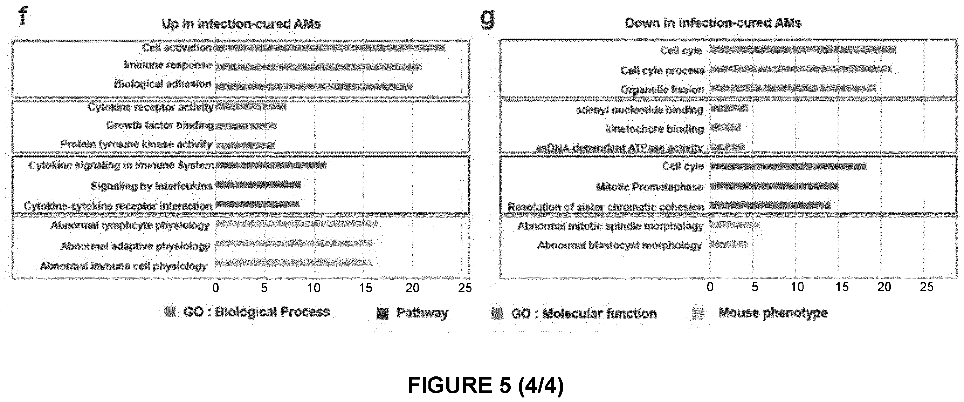

**Figure 5** represent a phenotypic analysis origin of paralyzed AM. Figure 5a is diagrams and histograms representing an analysis of expressions of classical markers (CD24, CD11b, CD64, CD11c, F4/80, FceR1a) on AM of (I) uninfected mice, or (II) 7 days after E. coli (infection cured) (n > 10 mice per group). Figure 5b is histogram representing the lactate production by AM (ordinate) of uninfected or infection-cured mice, after 24 hr of in vitro stimulation with RPMI or LPS (abscissa) (n=4 mice per group). Graphs represent mean ± SD and are pooled data from 2 independent experiments. Figure 5c is histogram representing TNF-α production (ordinate) by AM of uninfected mice, or 7 days E. coli pneumonia, after 24 hr stimulation with RPMI and LPS (abscissa) (n=4 mice per group). Graphs represent mean ± SD and are pooled data from 2 independent experiments. Figure 5d is a diagram of principal component (PC) analysis of uninfected AM (light gray) and infection-cured AM (dark gray). PC1 and PC2 show the percentage of variance explained for 12364 expressed genes. Figure 5e is a diagram of volcano plot of differential gene expression between infection-cured AM and uninfected AM. Significantly differentially expressed genes (log2FC >1.5 and -1.5) are shown in light gray. For complete results see Table 2_DEG. Figure 5 f-g are schema representing the gene Ontology analysis (Toppgene) of (f) upregulated or (g) downregulated genes in AM from infection-cured mice. Bar chart displays - log10 P-value. For complete results see Table 3.*p<0.05, **p<0.01

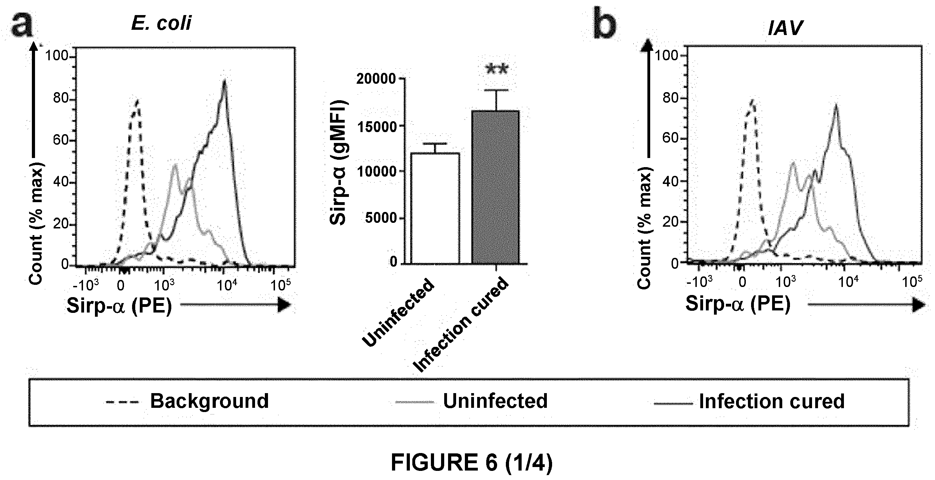

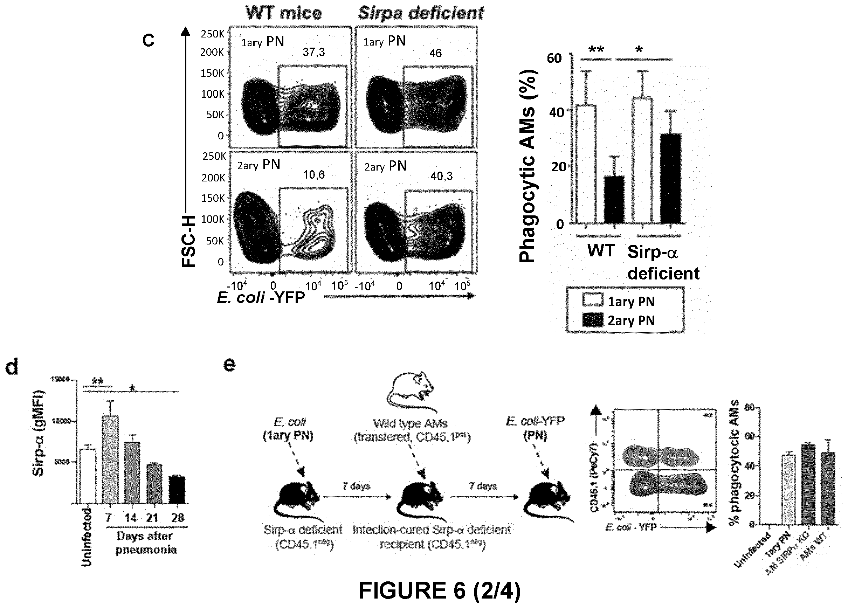

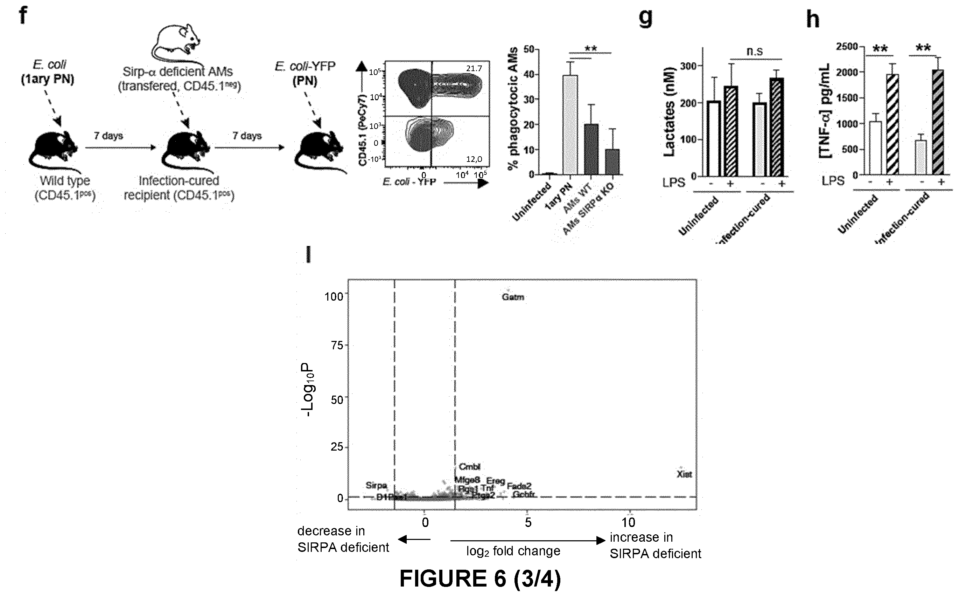

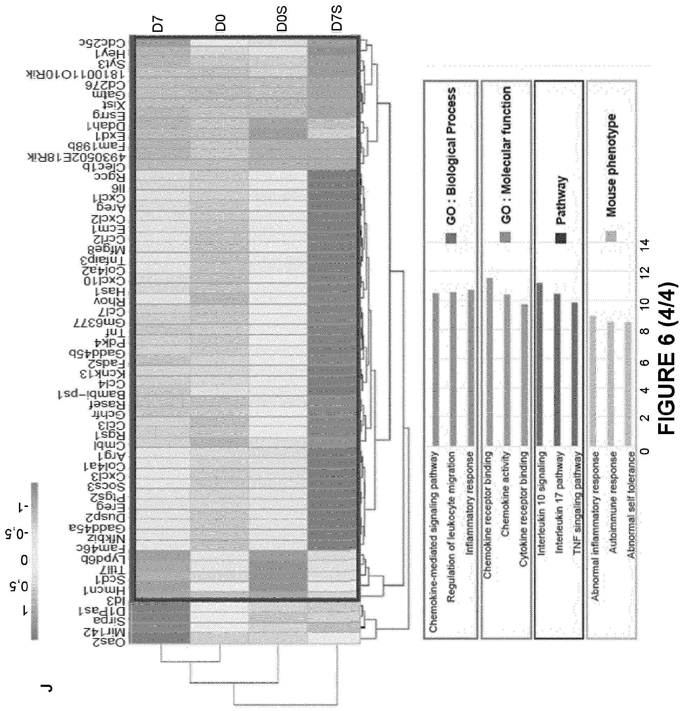

**Figure 6** demonstrates that SIRP-α is required for the priming, but not the maintenance, of the paralysis program of alveolar macrophages after infection. Figure 6 (a-b) SIRP-α expressions on AM of uninfected mice, or 7 days after E. coli (E. coli-infection cured) or 7 days after Influenza A Virus (IAV-infection cured). (n=4-8 mice per group). The histogram represent the Sirp-α (gMFI) (ordinate) in infected or infection-cured. Graphs represent mean ± SD and are pooled data from 2 independent experiments. Figure 6 c represent the percentages of phagocytic AM (ordinate) 2 hours after YFP-E. coli pneumonia in naive SIRP-α deficient mice (SHP2 knock-out) or littermate (1ary pneumonia) and in SIRP- α deficient (SHP2 knock-out) mice or littermate cured from E. coli pneumonia (2ary pneumonia) (abscissa). (n=5-8 mice per group). Graphs represent mean ± SD and are pooled data from 3 independent experiments. Figure 6 d represent a histogram of SIRP-α expression (gMFI) (ordinate) on AM of uninfected mice, or at the indicated time after E. coli (abscissa) (E. coli-infection cured. (n=2-3 mice per time point). Graphs represent mean ± SD and are pooled data from 1 experiment. Figure 6 e is a schema of the protocol: recipient SIRP-α deficient mice (SHP2 knock-out CD45.1neg) were infected with E. coli and 7 days later they were intra-tracheally injected with AM collected from uninfected wild type donors (CD45.1pos). 7 days later, recipients were injected with YFP-E. coli (i.t.) and the percentages of phagocytic cells were assessed on donor and recipient AM (n = 3-4 mice). The histogram represent the percentage of phagocytic of AM (ordinate) with regards to the uninfected, primary infected, SIRP-α deficient mice (CD45.1neg SHP2 knock-out) infected with E. coli or non deficient SIRP-α mice. Graphs represent mean ± SD from 1 experiment. Figure 6 f is a schema of the protocol: Recipient mice (CD45.1pos) were infected with E. coli and 7 days later they were intra-tracheally injected with AM collected from uninfected SIRP-α deficient donors (CD45.1neg SHP2 knock-out). 7 days later, recipients were injected with YFP-E. coli (i.t.) and the percentages of phagocytic cells were assessed on donor and recipient AM (n = 4-5 mice). The histogram represent the percentage of phagocytic of AM (ordinate) with regards to the uninfected, primary infected, SIRP-α deficient mice (CD45.1neg SHP2 knock-out) infected with E. coli or non deficient SIRP-α mice Graphs represent mean ± SD and are pooled data from 2 independent experiments. Figure 6 g is an histogram representing the lactate production in nM (ordinate) by AM of uninfected mice, or 7 days E. coli pneumonia, after 24 hr stimulation with RPMI and LPS (n=4 mice per group) (abscissa). Graphs represent mean ± SD and are pooled data from 2 independent experiments. Figure 6 h is an histogram representing the TNF-α production by AM in pg/mL (ordinate) of uninfected mice, or 7 days E. coli pneumonia, after 24 hr stimulation with RPMI and LPS (n=4 mice per group) (abscissa). Graphs represent mean ± SD and are pooled data from 2 independent experiments. Figure 6 i is a diagram representing a volcano plot of differential gene expression between infection-cured AM collected in Sirp-α deficient mice or in littermate. Significantly differentially expressed genes (log2FC >1.5 and -1.5) are shown in light gray. For complete results see Table 2. Figure 6 j represent a schema of gene Ontology analysis (Toppgene) of upregulated genes in AM from infection-cured Sirp-α deficient mice. Bar chart displays -log10 P-value. For complete results see Table 3.*p<0.05, **p<0.01, ***p<0.001

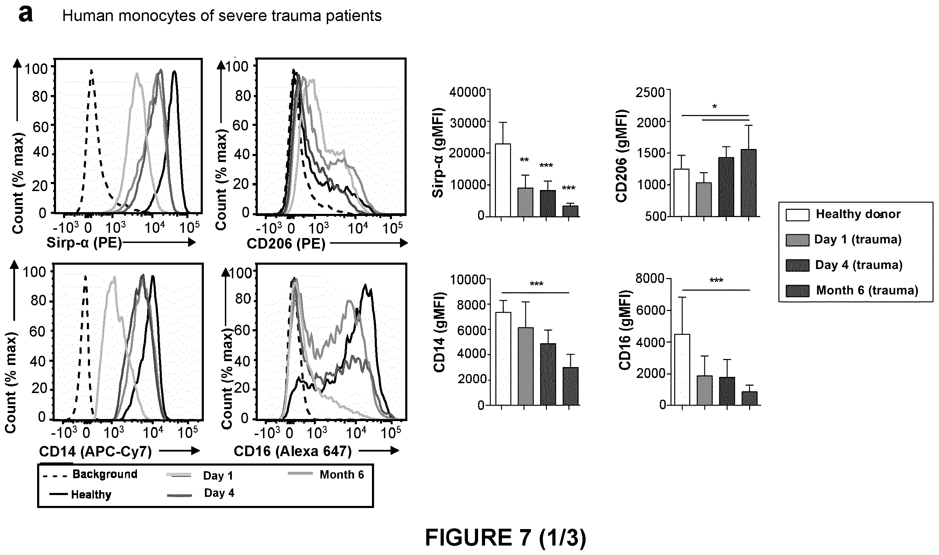

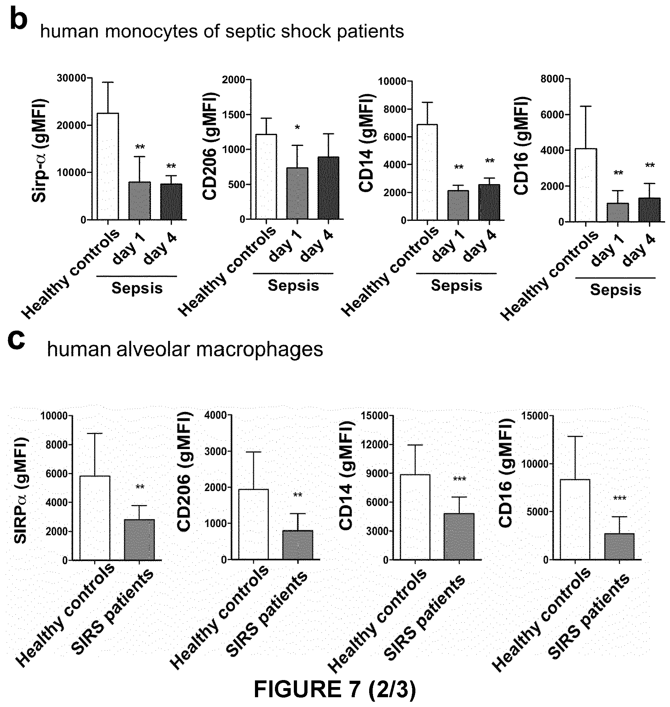

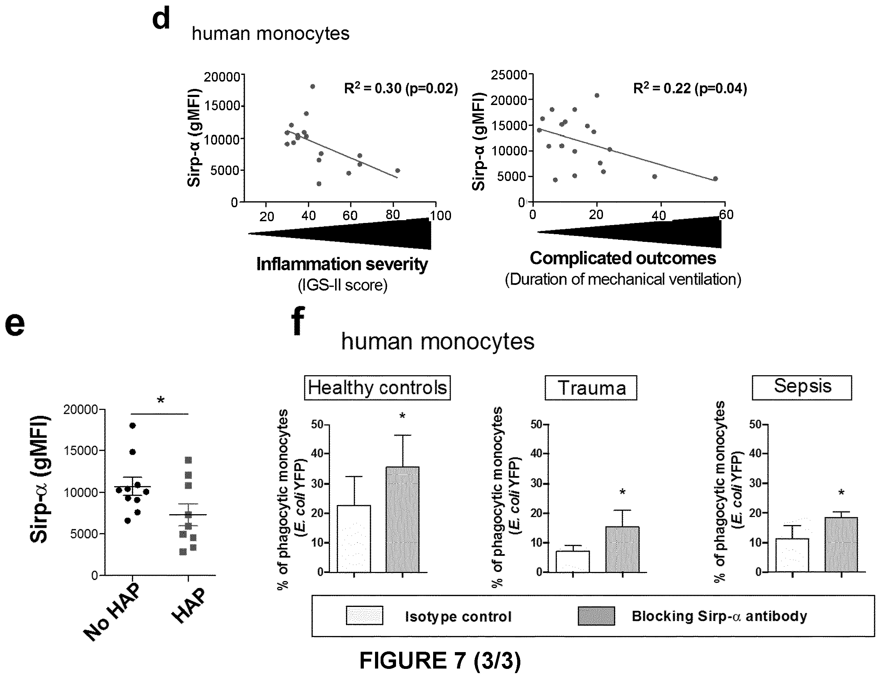

**Figure 7** demonstrates the potential of SIRP-α as biomarker and therapeutic target in human hospitalized patients at risk of secondary pneumonia. Figure 6 (a-c) are diagrams representing the expression of the inhibitory receptors SIRP- α of CD206, and of the activator receptors CD14 and of CD16, figure 6a represents schemas of expression on circulating monocytes from patients with post-traumatic systemic-inflammation (n=12-15 patients per group); figure 6 b represent histograms of expression on circulating monocytes from patients with sepsis-induced systemic-inflammation. (n=5-8 patients per group); and figure 6 c represent histograms of expression on AM from critically ill patients (n=8-10 patients per group). Graphs represent mean ± SD and are pooled data from 3 independent experiments. Figure 6 d represent a schema of correlation between the level of expression of SIRP-α at day 1 on circulating monocytes and inflammation severity (Apache II score) or complicated outcomes (duration of mechanical ventilation) in patients. Apache II rates the severity of critically ill patients from 15 (not critically ill) up to 144 (major risk of death). (n=19). Figure 6e is a diagram representing the expression of SIRP-α at day 1 on circulating monocytes in patients developing or not hospital-acquired pneumonia (HAP) during hospitalization (n=10 without HAP and n=9 patients with HAP). ). Figure 6f represents the percentage of phagocytic monocyte from healthy controls, trauma patients (day 1) and septic patients (day 1) which were cultured overnight with a blocking anti-human SIRP-α antibody (10µg/mL) then infected with YFP-E. coli. The percentages of phagocytic monocytes (CD14+) were measured at 1 hour after in vitro infection. (n=4-5 patients per group). Graphs represent mean ± SD and are pooled data from 3 independent experiments. *p<0.05, **p<0.01, ***p<0.001.

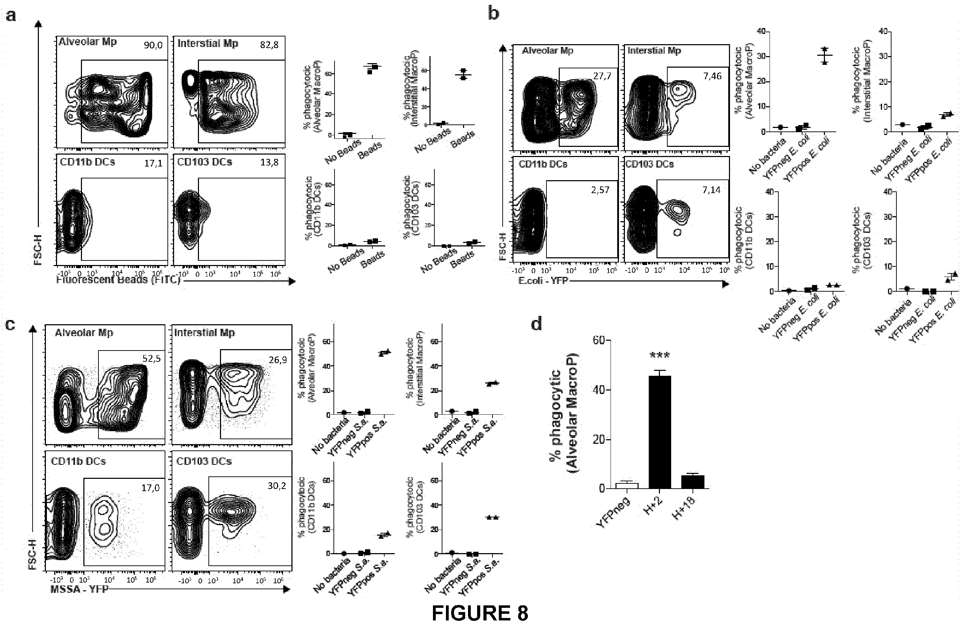

**Figure 8** demonstrate that alveolar macrophages are the main phagocytic cells of extracellular bacteria during acute pneumonia. Figure 8 (a) represent histograms of FITC alveolar macrophages, interstitial macrophages, CD11b dendritic cells and CD103 dendritic cells in presence or not of Fluoresbrite YG carboxylate microspheres (3.64×10⁹ beads, 0.5 mm; Polysciences) (abscissa) and the corresponding percentage of phagocytic cells (ordinate), Figure 8b are histograms obtained after YFP-Escherichia coli (E. coli, OD600=2-3, 75 µL),or Figure 8 c histograms obtained after YFP-Staphylococcus aureus (S.a., OD600=5-6, 75 µL), were injected i.t. in mice. 2 hours later, the percentages of FITC or YFP+ alveolar macrophages, interstitial macrophages, CD11b dendritic cells and CD103 dendritic cells in the lungs were measured by cytometry, the percentage of phagocytic cells (ordinate) was measured. Figure 8d represent a histogram showing the percentage of phagocytic AM (ordinate) two and 18 hours after the intra-tracheal instillation of YFP-E. coli, frequencies of YFP+ lung alveolar macrophages determined by flow cytometry analysis. n=2-3 mice per group. Graphs represent mean ± SD and are pooled data from 2 independent experiments.

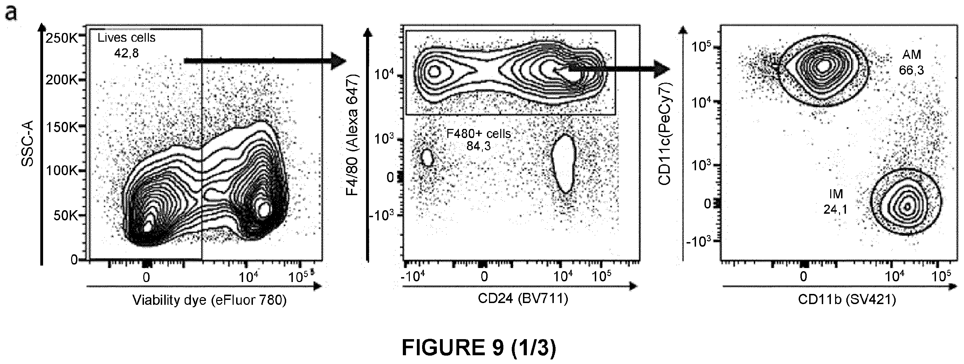

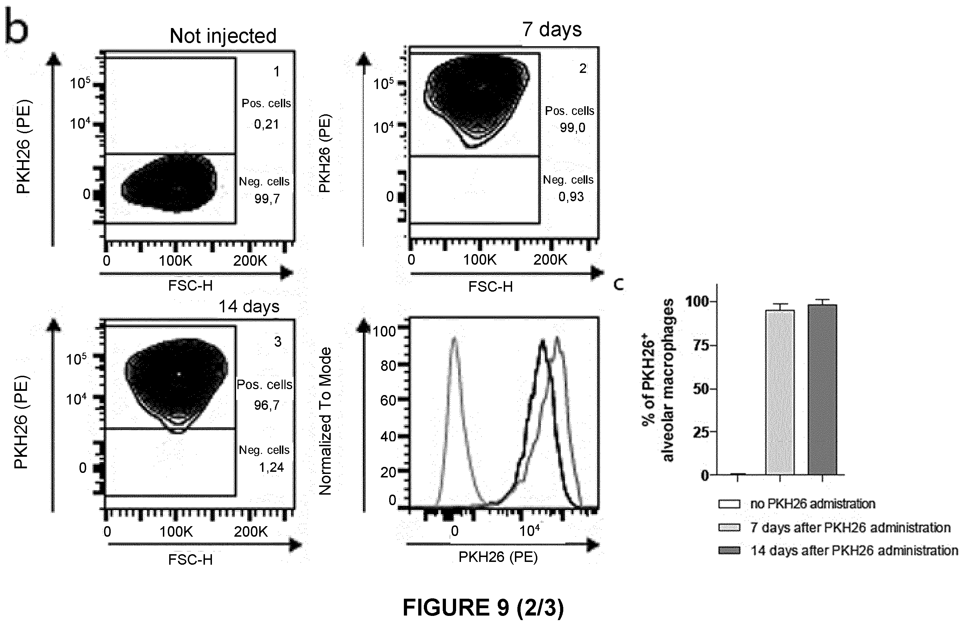

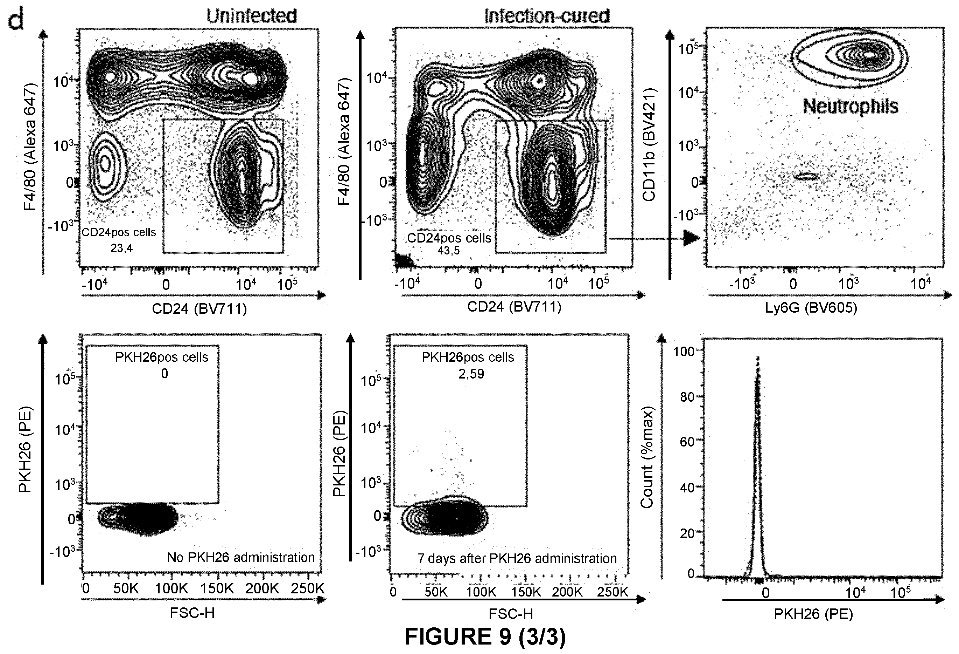

**Figure 9** represent long-lasting PKH26 labelling of tissue-resident alveolar macrophagesPKH26 (Red Fluorescent Phagocytic Cell Linker Kit; Sigma-Aldrich) was instilled directly into the lungs of mice (20 mM after dilution of Diluent B). Figure 9a is diagrams obtained from alveolar macrophages isolated by cytometry. Figure 9 b is diagrams of the percentages of resident (PKH26+) or recruited (PKH26-) was measured in mice not injected, or 7 or 14 days after injections, ordinate is PKH26 (PE), abscissa is FSC-H. Figure 9 c is a histogram of the percentages of resident (PKH26+) (ordinate) measured in mice not injected, or 7 or 14 days after injections (abscissa). Figure 9 d is diagram of PKH26 of lung neutrophils 7 days after injection in uninfected or infection-cured mice. ordinate is Count (% of max), abscissa is PKH26 (PE). n=2-3 mice per group. Graphs represent mean ± SD and are pooled data from 2 independent experiments.

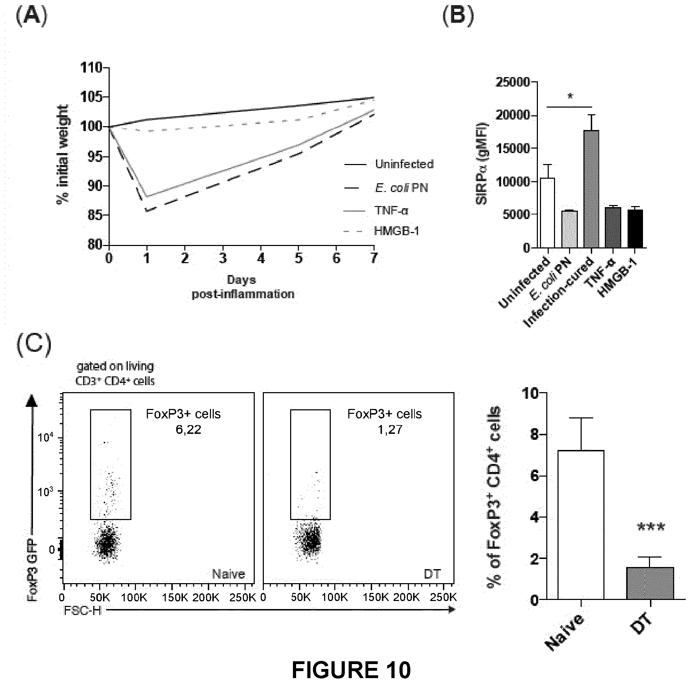

**Figure 10** represents the validations of TNF-α and HMGB1 tracheal instillation in wild type mice, and of Treg cell depletion in FoxP3-DTR mice. Figure 10a is a diagram representing the weight loss (ordinate) following intra-tracheal administration of E. coli (1ary PN) (broken line), of TNF-α (gray line) or of HMGB1 (dotted line). Figure 10b is an histogram of SIRP-α expression (ordinate) on AM of uninfected mice, or 7 days after tracheal instillation of E. coli (E. coli PN), or of TNF-α or of HMGB1 (abscissa). Figure 10c is diagrams and histogram of the percentage of FoxP3+CD4+ cells obtained after administration of Diphteria toxin (0.2 µg i.p, two injections 24 hours apart, then every 3 days) to DEREG mice to induce depletion of Treg cells respectively. DEREG mice were treated from day 4 after the primary pneumonia. Efficiency of depletion (number of cells) was controlled during experiments and routinely exceeded 90%. n=3-4 mice per group. *** p <0.001. Graphs represent mean ± SD and are pooled data from 2 independent experiments.

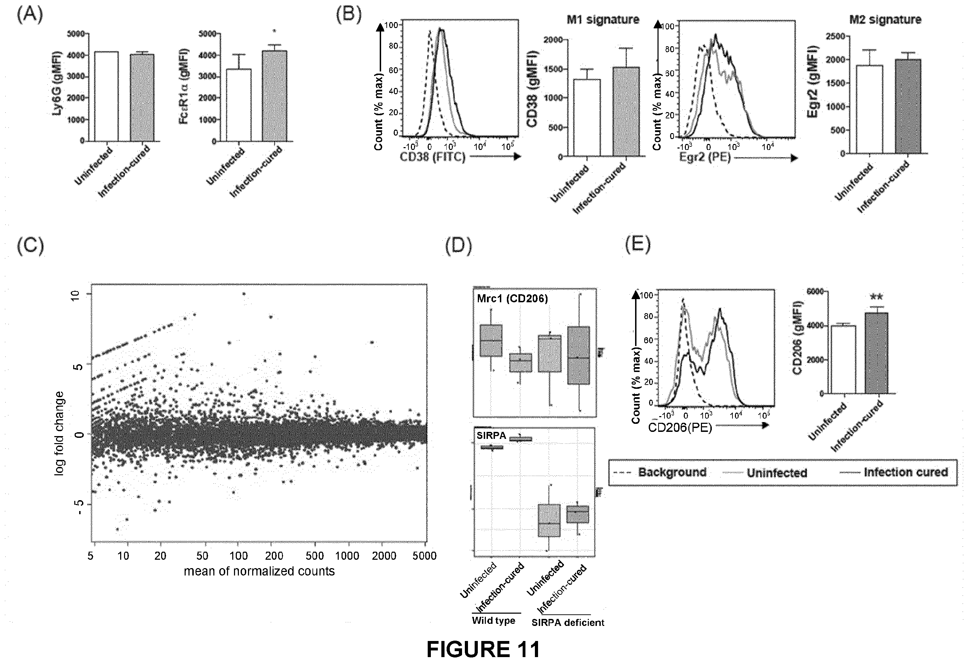

**Figure 11** represents the phenotype of AMs in infection-cured mice Figure 11A is histograms representing the expression of Ly6G and Mar1 (FcεR1α) in gMFI (ordinate) of AM in uninfected mice or 7 days after E. coli pneumonia (infection-cured mice) (abscissa). (n>10 mice/group). Figure 11b is diagrams and histograms representing the expression of CD38 and Egr2 in gMFI (ordinate) on AM of uninfected mice, or 7 days after E. coli (E. coli-infection cured) (abscissa).(n=6-8 mice per group). Graphs represent mean ± SD and are pooled data from 2 independent experiments Figure 11c is a diagram of MA-plot of gene expression from un-infected and 7day post infection AM. Genes shown in light gray are Benjamini-Hochberg (Q-value = 0.05) and absolute log2 fold change > 1.5). Figure 11d is histograms of mRNA level (ordinate) of the genes Mrc1 (up) or SIRPA (down) in AMs of uninfected or infection-cured wild type mice or SIRPA-deficient mice (abscissa) (n=3 mice per group). Figure 11e is histogram representing the expression of CD206 (Mrc1) in gMFI (ordinate) on AMs in uninfected or infection-cured mice (abscissa) (n=3-5 mice/group).

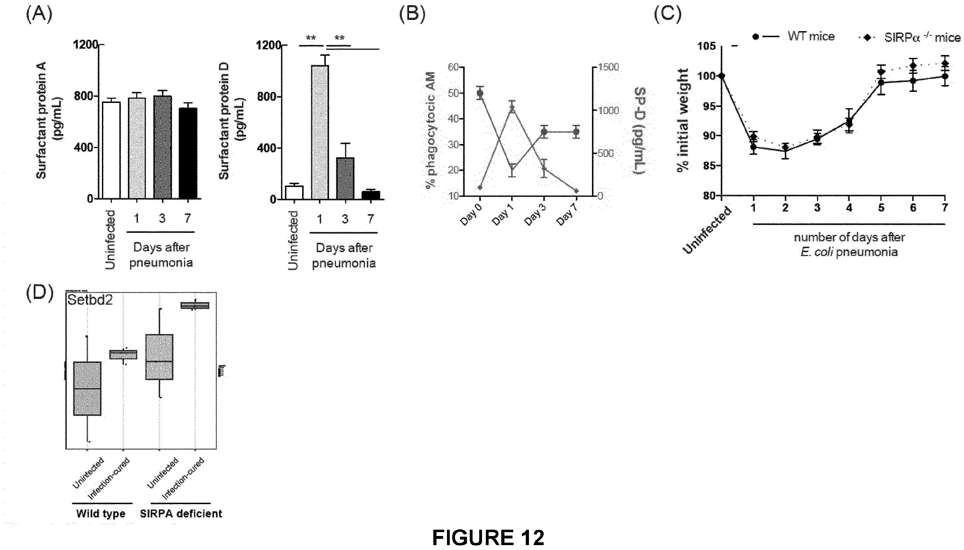

**Figure 12** represent results in primary pneumonia in wild type and in Sirp-α deficient mice, Figure 12a is histogram representing the concentration (pg/mL) of the surfactant proteins A and D in the bronchoalveolar fluid (ordinate) of mice at the indicated time in day (abscissa) after tracheal instillation of E. coli. Figure 12b represent the level of Surfactant Protein D (SP-D) and percentages of phagocytic AM 4 hours after YFP-E. Coli pneumonia after 90 minutes of prestimulation with lung homogenate collected at the indicated time of E. coli pneumonia. (n=3-4 mice per time point). Figure 12c is a diagram of weight loss (ordinate) following in days E. coli intra-tracheal administration in wild type (WT) and in SIRP-α deficient mice (dotted line) (n=4 mice per group). Figure 12d is an histogram representing the differential expression of Setbd2 mRNA (ordinate) in AM of wild type mice or SIRP-a deficient mice 7 days after E. coli pneumonia (n=2-3 mice per time point). (n=4 mice/group). Graphs represent mean ± SD and are pooled data from 2 independent experiments. *p<0.05, ** p< 0.01, ***p<0.001

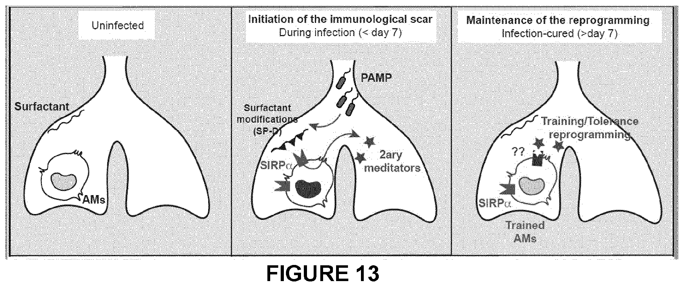

**Figure 13** is a schematic illustration of the time course of the training/tolerance reprogramming of AM by SIRP-α during and after pneumonia

### Equivalents

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

### Exemplification

The present invention and its applications can be understood further by the examples that illustrate some of the embodiments by which the inventive medical use may be reduced to practice. It will be appreciated, however, that these examples do not limit the invention. Variations of the invention, now known or further developed, are considered to fall within the scope of the present invention as described herein and as hereinafter claimed.

### Examples

### Example 1: effect of inhibitors on nosocomial disease and biological mechanism involved

### Material and methods

Mice used were C57BL/6J (B6), B6.SJL-PtprcaPep3b/BoyJ (CD45.1), C57BL/6J-Tlr9M7Btlr/Mmjax (Tlr9-/-)49, C57BL/6-Tg(Foxp3-DTR/EGFP)23.2Spar/Mmjax (Diphteria Toxin Receptor and GFP are expressed under the control of FoxP3 promoter, so-called DEREG mice)50, Tgfb2rfl/fl (Floxed regions around Tgfb2r gene)51 crossed to B6.Cg-Tg(Itgaxcre)1-1Reiz/J (in which Cre recombinase is expressed under the control of the CD11c promoter, so-called CD11 ccre mice)52, and SIRPαtm1Nog (SIRP-α-/-) mice53. For technical reasons, mice were used for experiments without taking gender into account. Male and female mice were maintained in specific pathogen-free conditions, group housed, at the Bio21 Institute Animal Facility (Parkville, Australia) or at the UTE-IRS2 Nantes Biotech Animal Facility (Nantes, France) following institutional guidelines and were used for experiments between six and fourteen weeks of age. Experimental procedures were approved by the Animal Ethics Committee of the University of Melbourne (protocol #1413066) and by the Animal Ethics Committee of the Pays de la Loire (APAFIS#7893-2015113011481071).

### Human subjects and human samples

Bioresources: IBIS-sepsis (severe septic patients) and IBIS (brain-injured patients), Nantes, France. Patients were enrolled from January 2016 to May 2017 in two French Surgical Intensive Care Units of one university hospital (Nantes, France). The collection of human samples has been declared to the French Ministry of Health (DC-2011-1399), and it has been approved by an institutional review board. Written informed consent from a next-of-kin was required for enrolment. Retrospective consent was obtained from patients, when possible.

For the IBIS-septic study, inclusion criteria were proven bacterial infection, together with a systemic inflammatory response (two signs or more among increased heart rate, abnormal body temperature, increased respiratory rate and abnormal white-cell count) and acute organ dysfunction and/or shock. For the IBIS study, inclusion criteria were brain-injury (Glasgow Coma Scale (GCS) below or equal to 12 and abnormal brain-CT scan) and systemic inflammatory response syndrome. Exclusion criteria were cancer in the previous five years, immunosuppressive drugs and pregnancy. All patients were clinically followed up for 28 days. Control samples were collected from matched healthy blood donors (age ± 10 years, sex, race), recruited at the Blood Transfusion Center (Etablissement Français du Sang, Nantes, France).

EDTA-anticoagulated blood samples were withdrawn 1 and 4 days after primary infection in septic patients (IBIS sepsis), or at day 1, day 4, and month 6 after ICU admission in brain-injured patients. Peripheral blood mononuclear cells (PBMCs) were isolated by centrifugation, frozen in liquid nitrogen in a 10% DMSO solution and stored until analysis.

Bioresources: IBIS-sepsis (severe septic patients) and IBIS (brain-injured patients), Nantes, France. Patients were enrolled from January 2014 to May 2016 in two French Surgical Intensive Care Units of one university hospital (Nantes, France). The collection of human samples has been declared to the French Ministry of Health (DC-2011-1399), and it has been approved by an institutional review board. Written informed consent from a next-of-kin was required for enrolment. Retrospective consent was obtained from patients, when possible.

For the IBIS-septic study, inclusion criteria were proven bacterial infection, together with a systemic inflammatory response (two signs or more among increased heart rate, abnormal body temperature, increased respiratory rate and abnormal white-cell count) and acute organ dysfunction and/or shock. For the IBIS study, inclusion criteria were brain-injury (Glasgow Coma Scale (GCS) below or equal to 12 and abnormal brain-CT scan) and systemic inflammatory response syndrome. Exclusion criteria were cancer in the previous five years, immunosuppressive drugs and pregnancy. All patients were clinically followed up for 28 days. Control samples were collected from matched healthy blood donors (age ± 10 years, sex, race), recruited at the Blood Transfusion Center (Etablissement Français du Sang, Nantes, France).

EDTA-anticoagulated blood samples were withdrawn seven days after primary infection in septic patients (IBIS sepsis), or at day 1 and day 7 ICU admission in trauma patients. Peripheral blood mononuclear cells (PBMCs) were isolated by centrifugation, frozen in liquid nitrogen in a 10% DMSO solution and stored until analysis.

### Induction of pneumonia

*Escherichia coli* (DH5α), grown for 18 hours in Luria broth medium at 37°C, was washed twice (1.000 × g, 10 min, 37°C), diluted in sterile isotonic saline and calibrated by nephelometry. *E.coli* (75 µL, OD₆₀₀=0.6-0.7) or Influenza virus (400 plaque-forming units of influenza, virus strain WSN x31) were injected intra-tracheally or intra-nasally respectively in anesthetized mice to induce a non-lethal acute pneumonia.

### Induction of aseptic lung inflammatory response

CpG 1668 (10 nM), TNF-α (2.5pg, Myltenyi Biotec, Paris, France) and HMGB1 (10µg, Elabscience, TX, USA) were administrated intra-tracheally under anaesthesia. Mice were kept in a semi-recumbent position for 60 seconds after injection.

### Cytokine, collectins, and lactate measurements.

Levels of TNF-α were measured with mouse TNF-α ELISA Ready-SET-Go kit (Thermo Fisher Scientific, MA, USA). Collectins SP-A and SP-D concentrations were determined with mouse surfactant associated protein A and D Ready-to-Use ELISA Kit from Didevelop (Jiangsu, China). Lactate assay kit (Sigma, St. Quentin Fallavier, France) was used to determine lactate levels.

### Treg cell depletion

Diphteria toxin (0.2 µg i.p, two injections 24 hours apart, then every 3 days) was administrated to DEREG mice to induce depletion of Treg cells respectively. DEREG mice were treated from day 4 after the primary pneumonia. Efficiency of depletion (number of cells) was controlled during experiments and routinely exceeded 90%.

### Generation of mixed bone marrow chimeras

Recipient mice were γ-irradiated twice with 550 Gray and were reconstituted with 2.5 - 5×10⁶ T cell-depleted bone marrow cells of each relevant donor strain at the indicated ratio. Neomycin (50 mg/ml) was added to the drinking water for the next 4 weeks. Chimeras were used for subsequent experiments 6 to 10 weeks after the reconstitution. Percentage of chimerism was tested during the experiments.

### Murine alveolar macrophage isolation and analysis

Macrophages purification from lungs and spleen, analytical and preparative flow cytometry were performed as described [17]. The following conjugated monoclonal antibodies were used: anti-CD11c (N418, BioLegend), anti-CD11b (M1/70, BD Biosciences), anti-CD24 (M1/69, BD Biosciences), anti-CD172a (SIRP-α, P84, BD Biosciences), anti-MHCII (M5/114, BioLegend), anti-CD45.1 (A20.1; eBioscience), anti-F4/80 (BM8, BioLegend), fixable Viability Dye (eBioscience). Samples were acquired on LSR-Fortessa or LSR-II (Becton Dickinson) and analyzed using Flowjo Software (TreeStar Inc, Ashland, OR). For adoptive transfers, AM were obtained from pooled bronchoalveolar lavages of 4-5 mice (purity >95%), and intra-tracheally injected in nonirradiated recipient (5-10.10⁴ cells / recipient).

### In vitro phagocytosis assay

Human PBMCs were thawed and cultured in complete media overnight with or without blocking anti-SIRP-α (OSE-172, humanized blocking anti-SIRPα clone18D5 10 µg.ml⁻¹, OSE Immunotherapeutics). PBMCs were washed twice then infected with YFP-E. *coli* (MOI of 1) or YFP-Methicillin Susceptible *Staphylococcus aureus* (MSSA) (MOI of 0.1) for 2 to 4 hours at 37.0°C. Monocytes were selected with an anti-human anti-CD14 antibody (63-D3, Biolegend). The frequencies of phagocytic monocytes were determined by flow cytometry (percentages of YFP⁺ cells among the CD14⁺ cells) at 1, 2 and 4 hours after the *in vitro* infection.

### In vivo phagocytic assay

YFP-S. *aureus* (Strain RN4220 with YFP/erm2 plasmid, kindly gifted by Malone, C. L. et al. 2009 [29]), and YFP-E. *coli* (strain DH5alpha with p-HG-1 plasmid, kindly gifted by Janssen, W. J. et al 2011 [30]) were grown overnight in Luria broth media with erythromycin 10 µg/mL or 20 µg/mL chloramphenicol, respectively. Fluoresbrite YG carboxylate microspheres (3.64 10⁹ beads, 0.5 mm; Polysciences), YFP-E. *coli* (OD₆₀₀=2-3, 75 µL) or YFP-S. *aureus* (OD₆₀₀=5-6, 75 µL) were injected i.t. in mice. 2 hours later, the percentages of FITC or YFP⁺ macrophages in the lungs were measured by flow cytometry.

### In vitro phagocytic assay for splenic macrophages

Splenocytes of uninfected mice or of infection-cured mice were infected with YFP-E. *coli* (MOI of 1) or YFP-Methicillin Susceptible *Staphylococcus aureus* (MSSA) (MOI of 0.1) for 2 hours at 37.0°C. Monocytes were selected with F4/80 and CD11b antibodies. The frequencies of phagocytic monocytes were determined by flow cytometry (percentages of YFP⁺ cells among the F4/80⁺ cells) 2 hours after the *in vitro* infection.

### ImageStream X assay (imaging flow cytometry).

Phagocytosis of YFP-E. *coli* was evaluated by ImageStreamX flow cytometer, which combines flow cytometry with confocal microscopy technology (ImageStream X Mark II, Amnis). Human monocytes (CD14⁺ cells) and murine AM (CD11C⁺ F4/80⁺ cells) images were acquired in the INSPIRE^{™} software on the ImageStreamX at 40X magnification, with excitation lasers 405 nm (120mW), 488 nm (20 mW), and 642 nm (150 mW). Data analysis was performed using the IDEAS software (Amnis Corporation). The gating strategy for analysis involved the selection of focused live cells first on viability marker, then on the fluorescence.

### Percentages of resident and of recruited alveolar macrophages in infection-cured mice

PKH26 (Red Fluorescent Phagocytic Cell Linker Kit; Sigma-Aldrich) was instilled directly into the lungs of mice (20 mM after dilution of Diluent B) (Urban, J. H. & Vogel, J. 2007 [31]. Mice were infected with *E*. *coli* (OD₆₀₀=2-3, 75 µL i.t.) at least 24 hours later after PKH26 to ensure selective labeling of resident alveolar macrophages. The percentages of resident (PKH26⁺) or recruited (PKH26⁻) alveolar macrophages were measured in BAL 7 days after infection.

### Intratracheal Transfer

For intratracheal transfer, 5-8×10⁵ AMs isolated from BAL in CD45.2⁺ mice were instilled directly into the lungs of anesthetized CD45.1⁺ animals. Phenotype and phagocytic function of the transferred cells was measured 7 days later as described above.

### BrDU incorporation

Mice were injected intraperitoneally with 1 mg bromodeoxyuridine (BrdU) (Sigma, St Louis, MO) at day 5 and at day 6 after pneumonia. At day 7, macrophages were isolated and analyzed as described (Kamath, A. T. et al. 2002, [32]).

### RNA-seq expression profiling

FACS sorted cells were lysed for RNA preparation using the QIAGEN RNEasy plus mini-kit. Three independent RNA samples were obtained from paralyzed AMs and from steady-state AMs in wild type and in SIRP-α knockout mice. One sample was removed from further analysis due to a low read counts (see table 4). FACS sorted cells were lysed for RNA preparation using the QIAGEN RNEasy plus mini-kit. Poly-A selected mRNA was then converted to Illumina sequencing ready libraries using NEB kit following the user-guide. cDNA libraries were pooled and sequenced at the Institut Cochin, Paris with one run of Illumina NextSeq 500. An average of 21 million 75bp single-end reads were obtained for each sample. Reads were mapped to mm 10 genome using STAR using default parameters (PMID: 23104886). The number of fragments mapped to each gene was counted using featureCounts and mm10.gtf gene annotation (Kamath, A. T. et al. 2002, [32]). The sequence data and read counts have been deposited to the EGA.

Differential expression analyses were performed using the DESeq2 package. Low abundance genes were filtered out leaving 12,329 genes for subsequent analysis. TMM normalization was used to adjust for under-sampling bias. Differential expressed genes were additionally filtered by an absolute log2 Fold-change of 1.5 while controlling the false discovery rate with the Benjamini-Hochberg method (5%). Gene ontology analysis was carried out using Toppgene suite (Chen, J., Bardes, E. E., Aronow, B. J. & Jegga, A. G. ToppGene Suite for gene list enrichment analysis and candidate gene prioritization. Nucleic Acids Res 37, W305-W311 (2009) [48])

### Statistical analysis

Data were plotted using GraphPad prism (La jolla, CA. United States). Unpaired T-test and Mann-Whitney unpaired test with two-tailed p-values and 95% confidence intervals. One-way ANOVA with Bonferonni correction (post-hoc tests) were used for multiple comparisons. Correlations were investigated by a linear regression test. Statistical details of experiments (exact number of mice per group, exact P-values, dispersion and precision measures) can be found in the figure legends. P < 0.05 for statistical significance.

### Results

### Protracted phagocytic defects in human monocytes after recovery from inflammation

Imaging flow cytometry was used measure the capacity of monocytes of trauma patients suffering systemic inflammation (table 1) to phagocytose extracellular bacteria expressing yellow fluorescent protein (YFP) (Figure 1a). This technique allowed to perform high-throughput quantitation of YFP^{pos} monocytes that harbored bacteria intracellularly (phagocytic) while distinguishing them from those that were YFP^{pos} but had bacteria simply adhered on the cell surface (non-phagocytic). The bacteria used were *Escherichia coli* (E. coli) and *Staphylococcus aureus* (S. aureus), the most frequent gram negative bacilli and gram positive cocci, respectively, responsible for HAP in severe septic and trauma patients. The percentage of phagocytic monocytes was lower in the patients, with no sign of recovery 6 months after the resolution of trauma-induced inflammation (Figure 1b-c). Phagocytosis was also reduced in monocytes of patients suffering severe sepsis (Figure 1d), showing this was a common feature in critically ill patients.

### Mouse alveolar macrophages newly produced after primary pneumonia are paralyzed

To test if phagocytic impairment was also observed in mouse phagocytes, a double-infection model was resorted to mimic the clinical scenario. Mice were first subjected to a bacterial (*E. coli*) or viral (*influenza A virus,* IAV) primary pneumonia, left to recover for seven days, and then infected intra-tracheally with fluorescent *E. coli* or *S. aureus* to cause secondary pneumonia (Figure 2a). The bacterial burden in these mice 24 h after infection was higher than in mice suffering primary pneumonia by the same pathogens (Figure 2b). In mice suffering primary pneumonia, AM were the most active cell type involved in phagocytosis of the bacteria, and the peak of phagocytosis was observed two hours after the infection (Figure 2c and Figure 8). The phagocytic activity of these AM was severely compromised during secondary pneumonia (Figure 2d-e) and remained significantly impaired for at least 28 days after the onset of primary pneumonia (Figure 2f). Persistent AM activation could not be the cause of impaired phagocytosis because we have previously reported that macrophages do not exhibit signs of activation 7 days after the primary infection12. Moreover, the rate of macrophage renewal in mice recovered from primary pneumonia, measured by BrDU incorporation, was comparable to that in non-infected mice (Figure 2g). This series of experiments demonstrate that AM, which continually turn-over in the lungs at a slow rate, develop with impaired capacity to capture bacteria for months after recovery from pneumonia.

### Paralyzed alveolar macrophages are derived from the resident macrophage population

The results above prompted us to characterize further the molecular mechanisms underpinning the functional shift of AM from highly phagocytic (before pneumonia) to poorly phagocytic (after clearing the primary infection). First, the signals responsible for reprogramming AM after inflammation acted systemically (potentially on bone marrow precursors) or locally were examined. The capacity of splenic macrophages to phagocytose *E. coli* or *S. aureus* was not altered 7 days after *E. coli pneumonia* (Figure 3a), indicating the immune defect triggered by the infection was local.

AM develop from fetal monocytes that differentiate into tissue-resident macrophages self-maintained locally throughout adult life, with minimal contribution from circulating monocytes. This process of renewal can also reconstitute macrophages lost in the course of less-severe infections (Roquilly, A. et al. 2016 [33], Hashimoto, D. et al. 2013 [34]). However, circulating monocytes might contribute to macrophage renewal following primary pneumonia if the severity of the disease led to extensive macrophage depletion, as this can open a niche for colonization by new monocyte-derived macrophages (Yao, Y. et al, 2018 [35], Kim, K.-W. et al. 2016 [36]). To determine whether the paralyzed AM observed after pneumonia were derived from local or external precursors, mice intra-tracheally with fluorescent dye PKH26 to label tissue-resident AM was instilled. Virtually all AM were labelled with this procedure (Figure 3b) and remained so in non-infected mice for at least 14 days (Figure 9a-c), confirming the low rate of replacement of this cell population in the absence of infection. Bacterial pneumonia with E. coli in mice instilled 24 hrs prior with PKH26, and analyzed the AM 7 days later. Most of these cells remained PKH26+, indicating they were the same cells that were labelled before the infection, or their progeny (Figure 3b). Neutrophils newly recruited to the lungs after infection were PKH26⁻ (Figure 9d), discarding the possibility that the dye lingered in the tissue and labelled AM derived from newly recruited, external precursors. The impaired AM observed after resolution of primary pneumonia are derived from a locally-renewing resident population.

### AM paralysis is maintained by endogenous signals that persist in the infected tissues

Paralysis was continually programmed by tissue signals, or by long-term modifications in the AM lineage. Intra-tracheally paralyzed AM collected from infection-cured mice (CD45.1^{neg}) into naive recipients (CD45.1^{pos}) was inoculated. These AM were functional 7 days after transfer (Figure 3c). Conversely, functional AM was inoculated from naive CD45.1^{neg} mice into CD45.1^{pos} infection-cured recipient mice, the donor AM became paralyzed (Figure 3d). Long-term maintenance of the paralysis program demonstrated that is dependent on signals remaining in the environment where infection occurred. Such signals might consist of endogenous mediators produced by the infected tissues (danger-associated molecular patterns or secondary inflammatory signals) (Van de Laar, L. et al, 2016 [37]) or by pathogen-associated molecular patterns that lingered at the infection site (Machiels, B. et al. 2017 [38]). This using mixed-bone marrow chimeras was addressed where irradiated wild-type (WT) mice were reconstituted with bone marrow from WT (CD45.1^{pos}) or Tlr9-/- (CD45.1^{neg}) donors in a 1:1 ratio. In this setting, the WT AM in the chimeric mice could respond directly to the pathogen-associated molecular pattern mimic CpG (recognized by TLR9), while Tlr9-/- AM could not recognize CpG but could respond to secondary signals produced by WT cells (Ma, K. C., et al. 2017 [39], Cegelski, L. et al. 2008 [40]) CpG was inoculated intra-tracheally in the chimeric animals and 7 days later infected them with E. coli-YFP to measure phagocytosis by WT and Tlr9^{-/-} AM (Figure 3e). Both groups of cells displayed impaired phagocytic function (Figure 3e), implying AM paralysis was induced not by direct encounter of pathogen products but by endogenous mediators. These mediators can be either inflammatory cytokines, such as Tumor Necrosis Factor (TNF)-α, or danger-associated molecular patterns (DAMP), such as high-mobility group box-1 (HMGB1), which are released in abundance during infection. To determine which of these two types of mediator is the main cause of the AM paralysis, mice was inoculated intratracheally with TNF-α or with HMGB1 and 7 days later infected them with E. coli-YFP. AM of TNF-α treated mice, but not those of HMGB1 treated mice, displayed impaired phagocytic function (Figure 3f and 10a-b), suggesting the former cytokine can be a cause, though not necessarily the only one, of AM paralysis induction.

### Treg cells and TGFβ are not major contributors to the paralysis program of AM

Other potential endogenous mediators were searched. Resolution of lung infections is accompanied with local accumulation of Tregs and TGF-β, two inhibitors of phagocytosis, so we addressed whether any of these two endogenous factors caused AM paralysis. Transgenic mice expressing the diphtheria toxin receptor (DTR) in FoxP3+ cells (DEREG mice) was infected, where Treg cells could be deplete (Figure 10c). Treg elimination during the resolution of primary pneumonia (from days 4 to 7 post primary infection) did not restore the capacity of AM to phagocytose E. coli or S. aureus during secondary pneumonia (Figure 4a). To assess a potential role for TGF-β, mixed-bone marrow chimeras was generated where recipient WT mice were reconstituted with a 3:1 ratio of two bone marrows: the first was WT and the second one TGFβR-II^{fl}/flCD11c^{cre}, which produces TGF-βRII-deficient AM (Figure 4b). Seven days after E. coli infection, the proportion of AM derived from the TGF-βR-deficient bone marrow (CD45.2+ cells) was significantly lower than in uninfected chimeras (Figure 4c), indicating that TGF-β promotes AM renewal after infection. However, these TGFβR-deficient AM were poorly phagocytic in infection-cured chimeras (Figure 4d). While these experiments do not discard a redundant role for Tregs or TGF-β, and perhaps other mediators, in AM paralysis induction, they illustrate that prevention of AM paralysis by targeting these mediators individually is not effective.

### Paralyzed AM does not display classical phenotype, but characteristics of training/tolerance reprogramming

To gain insights into the functional alterations that underpin AM paralysis, their expression of phenotypic markers and immunoregulatory factors were compared before and after pneumonia. Paralyzed AM expressed the characteristic surface markers of this cell type (F4/80, CD11c, CD11b, Ly6G, CD64, FcεR1α), but with significant changes in their expression levels (Figure 5a and Figure 11a). No changes were observed in markers that distinguish inflammatory M1 (CD38) from anti-inflammatory M2 (Egr2) macrophages (Figure 11b), suggesting that paralysis does not result from a protracted inflammatory response.

Pre-exposure of macrophages to microbes induces a state of training/tolerance reprogramming characterized by an increased metabolic activity and an alteration of the production of cytokines upon restimulation (Hussell, T. & Bell, T. J. 2014 [41], Barclay, A. N. 2009 [42], Li, L. X., et al. (2012) [43]). If the observed defect in phagocytosis was a specific phenomenon or a feature of a training/tolerance reprograming was questioned.

In AM from infection-cured mice the production of lactate was increased while the production of TNF-α was not decreased (Figure 5b-c). These results demonstrated that trained innate immunity can be associated a defect in phagocytosis capacity.

To compare more thoroughly the steady-state and paralyzed AM, out transcriptome analyses by RNA sequencing was carried out (see methods). Principal component analysis (PCA) of 12364 expressed genes in AM, showed that principal component 1 (PC1 = 85% of variance explained) separated steady-state AM from paralyzed AM suggesting that AM paralysis is a major contributor to transcriptional variation as compared to variation among conditions (PC2 =11%) (Figure 5d). To characterize the underlying gene expression changes differential expressed genes was calculated (DEG, see Methods) and found 247 upregulated genes and 141 down regulated genes in the paralyzed AMs compared to normally functional AM at a log2 fold change of 1.5. (Figure 5e, Figure 11c for MA-plot and Table 2 not shown).

Interestingly, the most differentially expressed gene, Cd163I1, encodes a group B scavenger receptor cysteine-rich protein involved in endocytosis, and which expression has been associated to an anti-inflammatory or anergic phenotype in macrophages (Lavin, Y. et al. 2014 [44], Amit, I., et al. 2015 [45]). To gain a more comprehensive overview of the genes altered, the DEG were analyzed for gene ontology (GO) enrichment (Figure 5f-g for representative GO and table 3 not shown). This analysis revealed that paralysis was associated with a transcriptional programming driving biological processes, including cellular activation and immune response as well as molecular functions of cytokine receptor activity and tyrosine kinase activity. This immune response activation was coupled to a down regulation of cell cycle genes, suggesting that the reprogramming affects function and proliferation of AM. This series of experiments demonstrate that E. coli pneumonia triggered a trained innate immunity programming lasting for weeks after removal of the trigger of AM and which combines decreased phagocytosis with increased cytokine receptor and tyrosine kinase activities.

### SIRP-α was required for the development of the paralysis programming

Among the genes differentially expressed in paralyzed AM, several encoded receptors or tyrosine kinase involved in regulation of phagocytosis. Some of these changes were validated by flow cytometry, and notably found a higher overall expression of SIRP-α, a regulator of tyrosine kinase-coupled signaling processes such as phagocytosis, on AM from mice infected-cured with E. *coli* or IAV (Figure 6a-b and Figure 11d-e), demonstrating this induction was not specific to infection with a particular pathogen.

Given the known inhibitory regulation of phagocytosis by SIRP-α, its involvement to the training of AM was searched. The time course during primary pneumonia of the concentrations of the surfactant proteins (SP)-A and D which are the most likely ligand of SIRP-α in the lungs was searched. The concentration of SP-A was not altered after E. *coli* pneumonia, surprisingly demonstrated found that the concentration of SP-D was negatively correlated with the inhibitory effect of lung homogenate and with the role of SIRP-α in the priming of the program (Figure 12a-b). The contribution of SIRP-α to phagocytosis impairment using mice deficient in this receptor. Weight loss during primary pneumonia and time to recovery was not altered in SIRP-α-deficient mice (Figure 12c), but AM phagocytosis during secondary pneumonia was highly improved (Figure 6c). Detailed analysis of SIRP-α expression on AM at different time-points after initiation of bacterial pneumonia showed an early increase followed by its reduction (Figure 6d), demonstrating different mechanisms for the initiation and for the maintenance of the paralysis program.

### SIRP-α modulates the suppressive microenvironment of AM after bacterial pneumonia

To test whether SIRP-α is required for the development of the paralysis program, intra-tracheally functional AM collected from wild type mice (CD45.1pos) were inoculated into infection-cured SIRP-α-deficient recipients (CD45.1neg). While normal AM transferred in wild type infection-cured mice became paralyzed (Figure 3d), AM transferred in SIRP-α deficient infection-cured mice did not become paralyze (Figure 6e). This result demonstrates that the local suppressive microenvironment does not develop in SIRP-α deficient mice. Functional AM collected from SIRP-α-deficient mice (CD45.1neg) were inoculated intra-tracheally into infection-cured wild type recipients (CD45.1pos). SIRP-α-deficient AM became paralyzed 7 days after transfer (Figure 6f). These experiments demonstrated that SIRP-α does not continuously inhibit the phagocytosis of newly formed AM, but triggers long lasting modifications of microenvironment involved in the local learning of phagocytosis by AM.

### SIRP-α deficiency alters the training/tolerance reprogramming of AM after infection

The impact of SIRP-α deficiency on the training/tolerance programming of AM was searched, and the metabolic and the cytokinic functions of AM in SIRP-α deficient were measured. Contrary to what we have observed in WT mice, the productions of lactate and of TNF-α of AM from infection-cured SIRP-α-deficient mice was not altered in after infection (Figure 6g-h). The RNA sequencing also showed that there were 59 genes differentially expressed (57 upregulated and 4 down regulated in SIRPαKO) in the trained WT AM compared to SIRP- α deficient AM, with absolute fold change above 1.5 (Fig. 6i). Interestingly, Xist, a long noncoding RNAs regulating of gene expression in immune cells37 and Setdb2 (Figure 12), a methyltransferase regulating chemokines response during viral pneumonia were amongst the most upregulated in the SIRP-α deficient cells compared to WT, suggesting a role for SIRP-α in the epigenetic modulation of AM after infection. Finally, GO analysis of SIRP-α upregulated genes include biological processes of chemokine-mediated signaling pathway, and chemokine activity associated to abnormal inflammatory response as well as autoimmune response (Figure 6j).

### Altered expression of regulators of phagocytosis in Systemic Inflammatory Response Syndrome patients

The results above demonstrate that acute inflammation causes prolonged defective phagocytosis in human monocytes and mouse macrophages (Figures 1 and 2).

The results also demonstrate that defective phagocytosis in mice is linked to altered expression of cell surface regulators of phagocytosis, the expression of similar molecules in human cells was analyzed. Circulating monocytes of severe trauma patients and of severe sepsis patients expressed altered levels of regulators of phagocytosis SIRP-α, CD206, CD14 and CD16 compared to cells from healthy controls (Figure 7a,b and Table 1 not shown for clinical description). Similar alterations were observed in AM contained in bronchoalveolar fluid from critically-ill patients suffering acute respiratory inflammation (Clinical Pulmonary Infection Score (CPIS) ≥ 639), compared to AM from patients requiring mechanical ventilation for scheduled surgery but not affected by inflammation ("healthy" controls) (Figure 7c and Table 1). Furthermore, the level of SIRP-α expression in circulating human monocytes correlated with the severity of inflammation and with the development of HAP in patients (Figure 7d-e). These results demonstrate that human AM and circulating monocytes change their expression of surface receptors involved in the regulation of phagocytosis, reproducing the observations of experimental sepsis in mice. These changes are accompanied with reduced capacity to phagocytose harmful bacteria (Figure 1). Induction of this state of paralysis is mediated by endogenous inflammatory processes (as they are manifest in trauma patients) and persist for long periods after the event that triggered severe inflammation.

### Blocking SIRP-α enhances phagocytosis by Systemic Inflammatory Response Syndrome patient cells

The SIRP-α inhibition as a therapeutic strategy to restore phagocytosis of extracellular bacteria by human paralyzed phagocytes was tested. Peripheral blood mononuclear cells from critically ill patients phagocytosed more E. coli-YFP in vitro in the presence of a blocking anti-SIRP-α antibody (Figure 7f). This demonstrates that while paralyzed monocytes showed reduced expression of SIRP-α, this receptor remained functional during the initial inflammatory response to infection.

### Discussion

As demonstrate above the inventors clearly demonstrate that:
(i) AM renew from local precursors following extracellular bacteria pneumonia;
(ii) the acquisition of phagocytic capacity in the developing AM is locally regulated by local tissue-derived signals;
(iii) such signals are in turn modulated by previous inflammatory responses to pathogens or cellular stress; SIRP-α playing a major role in these modifications, and
(iv) blocking SIRP-α antibody might represent a therapeutic target for host-targeted treatment of hospital-acquired infections. The months-long defects in bacterial uptake that we describe in mouse and human phagocytes following recovery from pneumonia or acute inflammation can be explained as the result of the maintenance of a paralysis program in the afflicted organ(s). Such program includes altered expression of regulatory receptors of phagocytosis. Inhibitors of such molecule, for example with mAb, restore phagocytic function and these molecules are diagnostic markers of susceptibility to secondary pneumonia.

While establishment of a low phagocytic environment post-infection may appear counterintuitive, there are other examples where dampening immune responsiveness is beneficial e.g. chronic viral infection (a form of "pathogen tolerance") and other situations where prevention of tissue damage and promotion of repair are more beneficial than persistent inflammation. The drawback of this mechanism is an increased susceptibility to extracellular bacteria in patients that survive sepsis or severe inflammation. Prevention or reversal of this "immune learning" program might protect against hospital-acquired infections following the primary insult.

The example clearly demonstrate that experimental pneumonia in mice, despite causing severe disease with significant bacterial load, did not lead to monocyte recruitment. It also demonstrate that AM were replenished from the pre-existing local pool but developed paralyzed. Importantly, the example demonstrate, in human suffering systemic inflammation, that the paralysis program alters circulating monocytes, supporting that this program is not lung specific but can develop in other organs affected, for example, by acute inflammation. As demonstrated above, inflammatory cytokines and DAMPs can alter SIRP-α expression (Figure 10b).

The example also clearly demonstrate that the paralysis program was not imprinted by the infection itself, as if that had been the case, AMs from infected mice would have developed into paralyzed progeny upon transfer into naive recipients. Rather, it was the environment left over by the infection that induced paralysis, which explains why AM from naive mice generated paralyzed AM upon transfer into infected mice. The example demonstrate that endogenous mediators, mainly inflammatory cytokines, are the causes of the paralysis program (Figure 3f-g).

Thus, the description of common "node" in the network of changes elicited by inflammation is an asset for the development of immunomodulators for patients at risk of secondary pneumonia. The finding that the complex phenomena of reprogramming can be simplified to some basic common outcomes (eg down-regulation of phagocytosis) allow to design innovative preventive approaches for secondary pneumonia. Moreover, as demonstrated above, this paralysis program in patients suffering from trauma-induced inflammation is mainly caused by DAMP or from sepsis-induced inflammation is caused by PAMP. The extrapolation of the role of SIRP-α in our mice model of secondary pneumonia to humans suffering from two different medical conditions further increases the potential extrapolation of these results to most of all the inflammatory conditions, and suggests that it can be a major node for the training/tolerogenic reprogramming of macrophages.

The example clearly demonstrate that the engagement of SIRP-α during bacterial pneumonia induces a specific training/tolerance program that shows high metabolic activity and yet poor phagocytosis. Importantly, the results demonstrate that the maintenance of the training status of AM required in vivo the maintenance of the local trained microenvironment.

The example demonstrate that SIRP-α initiates the trained/tolerance reprogramming of AMs which persists for months, and that once the microenvironment is altered the blockade of SIRP-α does not restore phagocytosis by AM. This observation support the fact that blocking SIRP-α could fail to restore phagocytosis when applied after the engagement of the SIRP-α intracellular pathways (Figure 13).

As demonstrated above, the invention allow to overcome the deficiency of AM cells, for example diminished capacity to phagocytose, and also to restore the immunity of a subject after an infection and thus allow to prevent or to treat secondary infection and/or nosocomial infection, with the administration inhibitors of SP-D and/or SP-D - SIRPα interaction and/or modulators of pathways/biological process for which SIRPα may be involved.

As demonstrated above, the invention allow to overcome the deficiency of AM cells, for example diminished capacity to phagocytose, and also to restore the immunity of a subject after an infection and thus allow to prevent or to treat secondary infection and/or nosocomial infection, with the administration inhibitors of SPH2 and/or inhibitor of the activation of SPH2 by SIRPα.

Accordingly, the inventor have clearly demonstrate that the present invention allows to treat secondary infection and/or nosocomial infection, in particular since the treatment is not directly directed to the pathogen or the cause of the disease but improve the defense of the treated subject.

The effects reported can be considered an extension of the phenomenon of "immunological training" induced locally by commensal flora and other environmental stimuli (Carr et al., 2016 [46]). The long term immunosuppression that ensues in mice or humans that survive severe infections can be considered a deleterious consequence of over-adaptation to a challenge that in normal conditions would lead to death but can be overcome in the controlled conditions of the laboratory (mice) or intensive care units (humans). Importantly, the inventors demonstrate that the signals that cause local cell imprinting are non-antigen specific, explaining why recovery from a primary infection can increase the susceptibility to an entirely new pathogen.

### List of References

1. Charlson et al., 2011, "Topographical continuity of bacterial populations in the healthy human respiratory tract." Am J Respir Crit Care Med. 2011 Oct 15;184(8):957-63. doi: 10.1164/rccm.201104-06550C. Epub 2011 Jun 16.
2. Ella Ott, Dr. med., et al. "The Prevalence of Nosocomial and Community Acquired Infections in a University Hospital An Observational Study Dtsch Arztebl Int. 2013 Aug; 110(31-32): 533-540
3. Mizgerd, Lung infection--a public health priority. PLoS Med. 2006 Feb;3(2):e76. Epub 2006 Jan 17.
4. Hotchkiss et al., 2013a
5. Roquilly and Villadangos, 2015
6. Bekaert, M. et al. Attributable Mortality of Ventilator-Associated Pneumonia. Am J Respir Crit Care Med 184, 1133-1139 (2011).
7. GBD 2015 DALYs and HALE Collaborators. Global, regional, and national disability-adjusted life-years (DALYs) for 315 diseases and injuries and healthy life expectancy (HALE), 1990-2015: a systematic analysis for the Global Burden of Disease Study 2015. Lancet 388, 1603-1658 (2016).
8. Eber, M. R., Laxminarayan, R., Perencevich, E. N. & Malani, A. Clinical and economic outcomes attributable to health care-associated sepsis and pneumonia. Arch. Intern. Med. 170, 347-353 (2010).
9. Torres, A. et al. International ERS/ESICM/ESCMID/ALAT guidelines for the management of hospital-acquired pneumonia and ventilator-associated pneumonia: Guidelines for the management of hospital-acquired pneumonia (HAP)/ventilator-associated pneumonia (VAP) of the European Respiratory Society (ERS), European Society of Intensive Care Medicine (ESICM), European Society of Clinical Microbiology and Infectious Diseases (ESCMID) and Asociación Latinoamericana del Tórax (ALAT). The European respiratory journal 50, (2017).
10. Kalil, A. C. et al. Management of Adults With Hospital-acquired and Ventilator-associated Pneumonia: 2016 Clinical Practice Guidelines by the Infectious Diseases Society of America and the American Thoracic Society. Clin. Infect. Dis. ciw353-51 (2016). doi: 10.1 093/cid/ciw353.
11. Klompas, M. The paradox of ventilator-associated pneumonia prevention measures. Crit Care 13, 315 (2009).
12. Weiss, E., Essaied, W., Adrie, C., Zahar, J.-R. & Timsit, J.-F. Treatment of severe hospital-acquired and ventilator-associated pneumonia: a systematic review of inclusion and judgment criteria used in randomized controlled trials. Crit Care 21, 162 (2017).
13.Asehnoune, K. et al. Hydrocortisone and fludrocortisone for prevention of hospital-acquired pneumonia in patients with severe traumatic brain injury (Corti-TC): a double-blind, multicentre phase 3, randomised placebo-controlled trial. Lancet Respir Med 2, 706-716 (2014).
14.Van Vught, L. A. et al. Incidence, Risk Factors, and Attributable Mortality of Secondary Infections in the Intensive Care Unit After Admission for Sepsis. JAMA 315, 1469-1479 (2016).
15. Belkaid, Y. & Harrison, O. J. Homeostatic Immunity and the Microbiota. Immunity 46, 562-576 (2017).
16. Charlson, E. S. et al. Topographical continuity of bacterial populations in the healthy human respiratory tract. Am J Respir Crit Care Med 184, 957-963 (2011).
17. Hotchkiss, R. S., Monneret, G. & Payen, D. Sepsis-induced immunosuppression: from cellular dysfunctions to immunotherapy. Nat Rev Immunol 13, 862-874 (2013).
18. Kishore U, Greenhough TJ, Waters P, Shrive AK, Ghai R, Kamran MF, et al. (2006). "Surfactant proteins SP-A and SP-D: structure, function and receptors". Mol Immunol. 43 (9): 1293-315. doi:10.1016/j.molimm.2005.08.004. PMID 16213021
19. Jens Madsen, Anette Kliem, Ida Tornrae, Karsten Skjradt, Claus Koch and Uffe Holmskov. Localization of Lung Surfactant Protein D on Mucosal Surfaces in Human Tissues. J Immunol 2000; 164:5866-5870; doi: 10.4049/jimmunol.164.11.5866. PMID:10820266
20. Leffleur et al. Production of human or humanized antibodies in mice. (2012). Production of human or humanized antibodies in mice, 901, 149-159. http://doi.org/10.1007/978-1-61779-931-0_9
21. Makoto Tanaka and Jonathan W Nyce. Respirable antisense oligonucleotides: a new drug class for respiratory disease. Respiratory Research 2000 2:2. https://doi.org/10.1186/rr32
22. Rongjun He, Li-Fan Zeng, Yantao He, Sheng Zhang, Zhong-Yin Zhang. Small molecule tools for functional interrogation of protein tyrosine phosphatases FEBS Journal 2013 https://dx.doi.org/10.1111/j.1742-4658.2012.08718.x
23.Ying-Nan P. Chen, Matthew J. LaMarche, Ho Man Chan, et al. Allosteric inhibition of SHP2 phosphatase inhibits cancers driven by receptor tyrosine kinases. Nature 2016, https://dx.doi.org/10.1038/nature18621
24. Sun, Lei, Wang et al. (Nature Communications, Engineered proteins with sensing and activating modules for automated reprogramming of cellular functions 2017 https://dx.doi.org/10.1038/s41467-017-00569-6
25. CELIA AITKEN et al. "Nosocomial Spread of Viral Disease" Clin Microbiol Rev. 2001 Jul; 14(3): 528-546
26. SCOTT K. FRIDKIN et al. "Epidemiology of Nosocomial Fungal Infection" Clin Microbiol Rev, 1996 ; 9(4): 499-511
27. Remington Pharmaceutical Sciences, sixteenth edition, E. W. Martin (Mack Publishing Co., Easton, Pa., 1980)
28. Marcel Geertz and Sebastian J. Maerkl, Experimental strategies for studying transcription factor-DNA binding specificities, Brief Funct Genomics. 2010 Dec; 9(5-6): 362-373
29. Malone, C. L. et al. Fluorescent reporters for Staphylococcus aureus. J. Microbiol. Methods 77, 251-260 (2009).
30. Janssen, W. J. et al. Fas determines differential fates of resident and recruited macrophages during resolution of acute lung injury. Am J Respir Crit Care Med 184, 547-560 (2011).
31. Urban, J. H. & Vogel, J. Translational control and target recognition by Escherichia coli small RNAs in vivo. Nucleic Acids Res. 35, 1018-1037 (2007).
32. Kamath, A. T., Henri, S., Battye, F., Tough, D. F. & Shortman, K. Developmental kinetics and lifespan of dendritic cells in mouse lymphoid organs. Blood 100, 1734-1741 (2002).
33. Roquilly, A. et al. Empiric antimicrobial therapy for ventilator-associated pneumonia after brain injury. Eur. Respir. J. 47, 1219-1228 (2016).
34. Hashimoto, D. et al. Tissue-Resident Macrophages Self-Maintain Locally throughout Adult Life with Minimal Contribution from Circulating Monocytes. Immunity 38, 792-804 (2013).
35.Yao, Y. et al. Induction of Autonomous Memory Alveolar Macrophages Requires T Cell Help and Is Critical to Trained Immunity. Cell 175, 1634-1650.e17 (2018).
36. Kim, K.-W., Zhang, N., Choi, K. & Randolph, G. J. Homegrown Macrophages. Immunity 45, 468-470 (2016).
37.Van de Laar, L. et al. Yolk Sac Macrophages, Fetal Liver, and Adult Monocytes Can Colonize an Empty Niche and Develop into Functional Tissue-Resident Macrophages. Immunity 44, 755-768 (2016).
38. Machiels, B. et al. A gammaherpesvirus provides protection against allergic asthma by inducing the replacement of resident alveolar macrophages with regulatory monocytes. Nat Immunol 18, 1310-1320 (2017).
39. Ma, K. C., Schenck, E. J., Pabon, M. A. & Choi, A. M. K. The Role of Danger Signals in the Pathogenesis and Perpetuation of Critical Illness. Am J Respir Crit Care Med (2017). doi:10.1164/rccm.201612-2460PP
40. Cegelski, L., Marshall, G. R., Eldridge, G. R. & Hultgren, S. J. The biology and future prospects of antivirulence therapies. Nat Rev Micro 6, 17-27 (2008).
41. Hussell, T. & Bell, T. J. Alveolar macrophages: plasticity in a tissue-specific context. Nat Rev Immunol 14, 81-93 (2014).
42. Barclay, A. N. Signal regulatory protein alpha (SIRPα)/CD47 interaction and function. Current Opinion in Immunology 21, 47-52 (2009).
43. Li, L. X., Atif, S. M., Schmiel, S. E., Lee, S. J. & McSorley, S. J. Increased Susceptibility to Salmonella Infection in Signal Regulatory Protein -Deficient Mice. The Journal of Immunology 189, 2537-2544 (2012).
44. Lavin, Y. et al. Tissue-Resident Macrophage Enhancer Landscapes Are Shaped by the Local Microenvironment. Cell 159, 1312-1326 (2014).
45.Amit, I., Winter, D. R. & Jung, S. The role of the local environment and epigenetics in shaping macrophage identity and their effect on tissue homeostasis. Nat Immunol 17, 18-25 (2015).
46. Carr EJ, Dooley J, Garcia-Perez J, Lagou V, Lee JC, Wouters C, Meyts I, Goris A, Boeckxstaens G, Linterman MA, Liston A. The cellular composition of the human immune system is shaped by age and cohabitation. Nat Immunol. 2016 Apr;17(4):461-468. doi: 10.1038/ni.3371. Epub 2016 Feb 15.
47. Linxiang Lan, Jane D Holland, Jingjing Qi, Stefanie Grosskopf, Regina Vogel, Balázs Györffy, Annika Wulf-Goldenberg, Walter Birchmeier Shp2 signaling suppresses senescence in PyMT-induced mammary gland cancer in mice The EMBO Journal 2015, 10.15252/embj.201489004
48. Chen, J., Bardes, E. E., Aronow, B. J. & Jegga, A. G. ToppGene Suite for gene list enrichment analysis and candidate gene prioritization. Nucleic Acids Res 37, W305-W311 (2009))

## Claims

1. Inhibitor of surface protein D (SP-D) for use in the prevention and/or treatment of secondary infection, wherein the inhibitor of surface protein (SP-D) is selected from anti-SP-D antibody, an anti-SP-D antibody fragment, a recombinant anti-SP-D antibody, a binding peptide, a siRNA, an antisense oligo or a ligand trap.

2. The inhibitor of surface protein (SP-D) for use according to claim 1 wherein the secondary infection is selected from the group comprising pneumonia, pleural infection, urinary infection, peritoneal infection, intra-abdominal abscess, meningitis, mediastinal infection and soft-tissue or skin infection.

3. The inhibitor of surface protein (SP-D) for use according to claim 1, wherein the binding peptide is LS-E15283.

4. The inhibitor of surface protein (SP-D) for use according to any of claims 1 to 3 wherein the inhibitor is used at a level from 0.1 to 100 µg.

5. The inhibitor of surface protein (SP-D) for use according to any of claims 1 to 4 wherein the inhibitor is administrated on single injection or repeated injections for up to 28 days.

6. Pharmaceutical composition comprising an inhibitor of surface protein (SP-D) for use in the prevention and/or treatment of a secondary infection wherein the inhibitor of surface protein (SP-D) is selected from anti-SP-D antibody, an anti-SP-D antibody fragment, a recombinant anti- SP-D antibody, a binding peptide, a siRNA, an antisense oligo, a ligand trap.

7. Pharmaceutical composition for use according to claim 6, wherein the inhibitor is at a level from 0.1 microg/kg to 1000 microg/kg per administration.

8. *Ex vivo* method for determining the susceptibility to a secondary disease of a subject comprising
a. Determining the concentration (Cₛ₁) and /or expression level (Lₛ₁) of surface protein D (SP-D) in a biological sample of said subject,
b. Comparison of the measured concentration Cₛ₁ and /or expression Lₛ₁ with a corresponding reference value C_{sref} and /or L_{sref} by calculating a score S3= Cₛ₁ / C_{sref} and/or S4= Lₛ₁ / L_{sref},
c. Determining the susceptibility to a secondary disease according to the value of score S3 and/or S4 obtained: If S3 > 1 and/or S4 > 1, the subject being considered likely to have a deficiency in immunity response to any pathogen, or, If S3 ≤ 1 and/or S4 ≤ 1, the subject being considered unlikely to have a deficiency in immunity response to any pathogen.

9. The method according to claim 8, wherein the biological sample is selected from the group consisting of a sample of tracheal fluid, bronchoalveolar lavages, and/or pleural fluid

10. The method according to claim 8 or 9, wherein the concentration of SP-D is determined with ELISA or RIA method.

11. The method according any of claim 8 to 10, wherein the reference value C_{sref} is from 1 pg/mL to 1000 mg/mL

12. The method according any of claim 8 to 10, wherein the reference value L_{sref} is from 1 pg/mL to 1000 mg/mL

13. Use of surface protein D (SP-D) as a biomarker of a secondary infection.

14. Inhibitor of SP-D/SIRPα interaction for use in the prevention and/or treatment of secondary infection wherein the inhibitor of SP-D/SIRPα interaction is selected from an anti-SIRPα antibody, an anti- SIRPα antibody fragment, a recombinant anti-SIRPα antibody, a binding peptide, a siRNA, an antisense oligo, a ligand trap or a small molecule SIRPα inhibitor.

15. The Inhibitor of SP-D/SIRPα interaction for use according to claim 14 wherein the small molecule inhibitor of SIRPα is NSC-87877

16. The Inhibitor of SP-D/SIRPα interaction for use according to claim 14 or 15 wherein the secondary infection is selected from the group comprising pneumonia, pleural infection, urinary infection, peritoneal infection, intra-abdominal abscess, meningitis, mediastinal infection and soft-tissue or skin infection.

17. The Inhibitor of SP-D/SIRPα interaction for use according to any of claims 14 to 16 wherein the inhibitor is used at a level from 0.1 to 100 µg.

18. The Inhibitor of SP-D/SIRPα interaction for use according to any of claims 14 to 17 wherein the inhibitor is administrated on single injection or repeated injections for up to 90 days.

19. Inhibitor of SHP-2 for use in the prevention and/or treatment of secondary infection wherein the inhibitor of SHP-2 is selected from an anti-SIRPα antibody, an anti- SIRPα antibody fragment, a recombinant anti-SIRPα antibody, a binding peptide, a siRNA, an antisense oligo, a ligand trap or a small molecule SIRPα inhibitor.

20. The inhibitor of SHP-2 for use according to claim 19 wherein the secondary infection is selected from the group comprising pneumonia, pleural infection, urinary infection, peritoneal infection, intra-abdominal abscess, meningitis, mediastinal infection and soft-tissue or skin infection.

21. The inhibitor of SHP-2 for use according to claims 19 or 20 wherein the inhibitor is used in doses from about 0.01 to 2000 mg, preferably from about 0.01 to 1000mg.

22. The inhibitor of SHP-2 for use according to any of claims 19 to 21 wherein the inhibitor is administrated on single injection or repeated injections for up to 90 days.

23. *Ex vivo* method for determining the susceptibility to a secondary disease of a subject comprising
a. Determining the concentration(Cₐ₁) and /or expression level (Lₐ₁) of SIRPα in a biological sample of said subject,
b. Comparison of the measured concentration Cₐ₁ and /or expression Lₐ₁ with a corresponding reference value C_{aref} and /or L_{aref} by calculating a score S5= Cₐ₁/ C_{aref} and/or S6= Lₐ₁/L_{aref},
c. Determining the susceptibility to a secondary disease according to the value of score S5 and/or S6 obtained: If S5 > 1 and/or S6 > 1 , the subject being considered likely to have a deficiency in immunity response to any pathogen, or, If S5 ≤ 1 and/or S6 ≤ 1, the subject being considered unlikely to have a deficiency in immunity response to any pathogen.

24. The method according to claim 23, wherein the biological sample is selected from the group consisting of a sample of tracheal fluid, bronchoalveolar lavages, pleural fluid and/or circulating leukocytes.

25. The method according any of claim 23 to 24, wherein the reference value C_{aref} is from 1 pg/mL to 100 mg/mL.

26. The method according any of claim 23 to 25, wherein the reference value L_{aref} is from 0 to 100 ratio of house-keeping gene (PCR) or from 10 to 80% positive cells (flow cytometry)

27. Use of surface SIRPα as a biomarker of a secondary infection.

## Patentansprüche

1. Inhibitor eines Oberflächenproteins D (SP-D) zur Verwendung bei der Vorbeugung und/oder der Behandlung von Sekundärinfektionen, wobei der Inhibitor des Oberflächenproteins (SP-D) aus einem anti-SP-D-Antikörper, einem anti-SP-D-Antikörperfragment, einem rekombinanten anti-SP-D-Antikörper, einem Bindungspeptid, einer siRNA, einem Antisense-Oligo oder einer Ligandenfalle ausgewählt ist.

2. Inhibitor des Oberflächenproteins (SP-D) zur Verwendung nach Anspruch 1, wobei die Sekundärinfektion aus der Gruppe ausgewählt ist, umfassend Pneumonie, Pleurainfektion, Harninfektion, Peritonealinfektion, intraabdominalen Abszess, Meningitis, Mediastinalinfektion und Weichgewebe- oder Hautinfektion.

3. Inhibitor des Oberflächenproteins (SP-D) zur Verwendung nach Anspruch 1, wobei das Bindungspeptid LS-E15283 ist.

4. Inhibitor des Oberflächenproteins (SP-D) zur Verwendung nach einem der Ansprüche 1 bis 3, wobei der Inhibitor bei einem Niveau von 0,1 bis 100 µg verwendet wird.

5. Inhibitor des Oberflächenproteins (SP-D) zur Verwendung nach einem der Ansprüche 1 bis 4, wobei der Inhibitor in einer einzelnen Injektion oder wiederholten Injektionen für bis zu 28 Tage verabreicht wird.

6. Pharmazeutische Zusammensetzung, umfassend einen Inhibitor des Oberflächenproteins (SP-D) zur Verwendung bei der Vorbeugung und/oder der Behandlung einer Sekundärinfektion, wobei der Inhibitor des Oberflächenproteins (SP-D) aus einem anti-SP-D-Antikörper, einem anti-SP-D-Antikörperfragment, einem rekombinanten anti-SP-D-Antikörper, einem Bindungspeptid, einer siRNA einem Antisense-Oligo, einer Ligandenfalle ausgewählt ist.

7. Pharmazeutische Zusammensetzung zur Verwendung nach Anspruch 6, wobei der Inhibitor pro Verabreichung auf einem Niveau von 0,1 Mikrog/kg bis 1000 Mikrog/kg liegt.

8. Ex-vivo-Verfahren zum Bestimmen der Anfälligkeit für eine Sekundärerkrankung eines Subjekts, umfassend
a. Bestimmen der Konzentration (Cₛ₁) und/oder des Expressionsniveaus (Lₛ₁) an Oberflächenprotein D (SP-D) in einer biologischen Probe des Subjekts,
b. Vergleich der gemessenen Konzentration Cₛ₁ und/oder der Expression Lₛ₁ mit einem entsprechenden Referenzwert C_{sref} und/oder L_{sref} durch Berechnen einer Bewertungsbewertung S3= Cₛ₁ / C_{sref} und/oder S4= Lₛ₁ / L _{sref},
c. Bestimmen der Anfälligkeit für eine Sekundärerkrankung gemäß dem Wert von Bewertung S3 und/oder S4, der erhalten wird: Falls S3 > 1 und/oder S4 > 1, als wahrscheinlich angesehen wird, dass das Subjekt einen Mangel an Immunantwort gegenüber einem beliebigen Pathogen aufweist, oder, Falls S3 ≤ 1 und/oder S4 ≤ 1, als unwahrscheinlich angesehen wird, dass das Subjekt einen Mangel an Immunantwort gegenüber einem beliebigen Pathogen aufweist.

9. Verfahren nach Anspruch 8, wobei die biologische Probe aus der Gruppe ausgewählt ist, bestehend aus einer Probe von Trachealfluid, bronchoalveolären Lavage und/oder Pleurafluid

10. Verfahren nach Anspruch 8 oder 9, wobei die Konzentration von SP-D mit einem ELISA- oder RIA-Verfahren bestimmt wird.

11. Verfahren nach einem der Ansprüche 8 bis 10, wobei der Referenzwert C_{sref} von 1 pg/mL bis 1000 mg/mL beträgt

12. Verfahren nach einem der Ansprüche 8 bis 10, wobei der Referenzwert L_{sref} von 1 pg/mL bis 1000 mg/mL beträgt

13. Verwendung des Oberflächenproteins D (SP-D) als ein Biomarker einer Sekundärinfektion.

14. Inhibitor der SP-D/SIRPα-Interaktion zur Verwendung bei der Vorbeugung und/oder der Behandlung von Sekundärinfektionen, wobei der Inhibitor der SP-D-/SIRPα-Interaktion aus einem anti-SIRPα-Antikörper, einem anti-SIRPα-Antikörperfragment, einem rekombinanten anti-SIRPα-Antikörper, einem Bindungspeptid, einer siRNA, einem Antisense-Oligo, einer Ligandenfalle oder einem SIRPα-Inhibitor eines kleinen Moleküls ausgewählt ist.

15. Inhibitor der SP-D-/SIRPα-Interaktion zur Verwendung nach Anspruch 14, wobei der Inhibitor des kleinen Moleküls des SIRPα NSC-87877 ist

16. Inhibitor der SP-D-/SIRPα-Interaktion zur Verwendung nach Anspruch 14 oder 15, wobei die Sekundärinfektion aus der Gruppe ausgewählt ist, umfassend Pneumonie, Pleurainfektion, Harninfektion, Peritonealinfektion, intraabdominalen Abszess, Meningitis, Mediastinalinfektion und Weichgewebe- oder Hautinfektion.

17. Inhibitor der SP-D-/SIRPα-Interaktion zur Verwendung nach einem der Ansprüche 14 bis 16, wobei der Inhibitor auf einem Niveau von 0,1 bis 100 µg verwendet wird.

18. Inhibitor der SP-D-/SIRPα-Interaktion zur Verwendung nach einem der Ansprüche 14 bis 17, wobei der Inhibitor in der einzelnen Injektion oder wiederholten Injektionen für bis zu 90 Tage verabreicht wird.

19. Inhibitor von SHP-2 zur Verwendung bei der Vorbeugung und/oder der Behandlung von Sekundärinfektionen, wobei der Inhibitor von SHP-2 aus einem anti-SIRPα-Antikörper, einem anti-SIRPα-Antikörperfragment, einem rekombinanten anti-SIRPα-Antikörper, einem Bindungspeptid, einer siRNA, einem Antisense-Oligo, einer Ligandenfalle oder einem SIRPα-Inhibitor des kleinen Moleküls ausgewählt ist.

20. Inhibitor von SHP-2 zur Verwendung nach Anspruch 19, wobei die Sekundärinfektion aus der Gruppe ausgewählt ist, umfassend Pneumonie, Pleurainfektion, Harninfektion, Peritonealinfektion, intraabdominalen Abszess, Meningitis, Mediastinalinfektion und Weichgewebe- oder Hautinfektion.

21. Inhibitor von SHP-2 zur Verwendung nach den Ansprüchen 19 oder 20, wobei der Inhibitor in Dosen von etwa 0,01 bis 2000 mg, vorzugsweise von etwa 0,01 bis 1000 mg, verwendet wird.

22. Inhibitor von SHP-2 zur Verwendung nach einem der Ansprüche 19 bis 21, wobei der Inhibitor in der einzelnen Injektion oder wiederholten Injektionen für bis zu 90 Tage verabreicht wird.

23. Ex-vivo-Verfahren zum Bestimmen der Anfälligkeit für eine Sekundärerkrankung eines Subjekts, umfassend
a. Bestimmen der Konzentration (Ca1) und/oder des Expressionsniveaus (La1) von SIRPα in einer biologischen Probe des Subjekts,
b. Vergleich der gemessenen Konzentration Ca1 und/oder der Expression La1 mit einem entsprechenden Referenzwert Caref und/oder Laref durch Berechnen einer Bewertung S5= Ca1/ Caref und/oder S6= La1/Laref,
c. Bestimmen der Anfälligkeit für eine Sekundärerkrankung gemäß dem Wert von Bewertung S5 und/oder S6, die erhalten wird: Falls S5 > 1 und/oder S6 > 1, als wahrscheinlich angesehen wird, dass das Subjekt einen Mangel an Immunantwort gegenüber einem beliebigen Pathogen aufweist, oder, Falls S5 ≤ 1 und/oder S6 ≤ 1, als unwahrscheinlich angesehen wird, dass das Subjekt einen Mangel an Immunantwort gegenüber einem beliebigen Pathogen aufweist.

24. Verfahren nach Anspruch 23, wobei die biologische Probe aus der Gruppe ausgewählt ist, bestehend aus einer Probe von Trachealfluid, bronchoalveolären Lavage, Pleurafluid und/oder zirkulierenden Leukozyten.

25. Verfahren nach einem der Ansprüche 23 bis 24, wobei der Referenzwert C_{aref} 1 pg/mL bis 100 mg/mL beträgt.

26. Verfahren nach einem der Ansprüche 23 bis 25, wobei der Referenzwert L_{aref} von 0 bis 100-Verhältnis eines Haushaltgens (PCR) oder von 10 bis 80 % positive Zellen (Durchflusszytometrie) beträgt

27. Verwendung von Oberflächen-SIRPα als ein Biomarker einer Sekundärinfektion.

## Revendications

1. Inhibiteur de la protéine de surface D (SP-D) pour utilisation dans la prévention et/ou le traitement d'une infection secondaire, dans lequel l'inhibiteur de la protéine de surface (SP-D) est choisi parmi un anticorps anti-SP-D, un fragment d'anticorps anti-SP-D, un anticorps recombinant anti-SP-D, un peptide de liaison, un siRNA, un oligo antisens ou un piège à ligands.

2. Inhibiteur de la protéine de surface (SP-D) pour utilisation selon la revendication 1, dans laquelle l'infection secondaire est choisie dans le groupe comprenant la pneumonie, l'infection pleurale, l'infection urinaire, l'infection péritonéale, l'abcès intra-abdominal, la méningite, l'infection médiastinale et l'infection des tissus mous ou de la peau.

3. Inhibiteur de la protéine de surface (SP-D) pour utilisation selon la revendication 1, dans laquelle le peptide de liaison est LS-E15283.

4. Inhibiteur de la protéine de surface (SP-D) pour utilisation selon l'une des revendications 1 à 3, dans lequel l'inhibiteur est utilisé à un niveau de 0,1 à 100 µg.

5. Inhibiteur de la protéine de surface (SP-D) pour utilisation selon l'une des revendications 1 à 4, dans lequel l'inhibiteur est administré par injection unique ou par injections répétées pendant 28 jours au maximum.

6. Composition pharmaceutique comprenant un inhibiteur de la protéine de surface (SP-D) pour utilisation dans la prévention et/ou le traitement d'une infection secondaire, l'inhibiteur de la protéine de surface (SP-D) étant choisi parmi un anticorps anti-SP-D, un fragment d'anticorps anti-SP-D, un anticorps recombinant anti-SP-D, un peptide de liaison, un siRNA, un oligo antisens, un piège à ligands.

7. Composition pharmaceutique selon la revendication 6, dans laquelle l'inhibiteur est à un niveau de 0,1 microg/kg à 1000 microg/kg par administration.

8. Méthode ex *vivo* pour déterminer la susceptibilité d'un sujet à une maladie secondaire comprenant
a. Déterminer la concentration (Cₛ₁) et/ou le niveau d'expression (Lₛ₁) de la protéine de surface D (SP-D) dans un échantillon biologique dudit sujet,
b. Comparaison de la concentration mesurée Cₛ₁ et/ou de l'expression Lₛ₁ avec une valeur de référence correspondante C_{sref} et/ou L_{sref} par le calcul d'un score S3= Cₛ₁ / C_{sref} et/ou S4= Lₛ₁ / L_{sref} ,
c. Déterminer la susceptibilité à une maladie secondaire en fonction de la valeur du score S3 et/ou S4 obtenu : Si S3 > 1 et/ou S4 > 1, le sujet étant considéré comme susceptible de présenter un déficit de la réponse immunitaire vis-à-vis d'un agent pathogène quelconque, ou, Si S3 ≤ 1 et/ou S4 ≤ 1, le sujet étant considéré comme peu susceptible de présenter un déficit de la réponse immunitaire vis-à-vis de tout pathogène.

9. Méthode selon la revendication 8, dans laquelle l'échantillon biologique est choisi dans le groupe constitué d'un échantillon de liquide trachéal, de lavages broncho-alvéolaires et/ou de liquide pleural.

10. Méthode selon la revendication 8 ou 9, dans laquelle la concentration de SP-D est déterminée par une méthode ELISA ou RIA.

11. Méthode selon l'une des revendications 8 à 10, dans laquelle la valeur de référence C_{sref} est comprise entre 1 pg/mL et 1000 mg/mL.

12. Méthode selon l'une des revendications 8 à 10, dans laquelle la valeur de référence L_{sref} est comprise entre 1 pg/mL et 1000 mg/mL.

13. Utilisation de la protéine de surface D (SP-D) comme biomarqueur d'une infection secondaire.

14. Inhibiteur de l'interaction SP-D/SIRPα pour utilisation dans la prévention et/ou le traitement d'une infection secondaire, l'inhibiteur de l'interaction SP-D/SIRPα étant choisi parmi un anticorps anti-SIRPα, un fragment d'anticorps anti-SIRPα, un anticorps recombinant anti-SIRPα, un peptide de liaison, un siRNA, un oligo antisens, un piège à ligands ou une petite molécule inhibiteur de SIRPα.

15. Inhibiteur de l'interaction SP-D/SIRPα pour utilisation selon la revendication 14, dans laquelle la petite molécule inhibitrice de SIRPα est le NSC-87877.

16. Inhibiteur de l'interaction SP-D/SIRPα utilisé selon la revendication 14 ou 15, dans lequel l'infection secondaire est choisie dans le groupe comprenant la pneumonie, l'infection pleurale, l'infection urinaire, l'infection péritonéale, l'abcès intra-abdominal, la méningite, l'infection médiastinale et l'infection des tissus mous ou de la peau.

17. Inhibiteur de l'interaction SP-D/SIRPα pour utilisation selon l'une des revendications 14 à 16, dans lequel l'inhibiteur est utilisé à un niveau compris entre 0,1 et 100 µg.

18. Inhibiteur de l'interaction SP-D/SIRPα pour utilisation selon l'une des revendications 14 à 17, dans laquelle l'inhibiteur est administré par injection unique ou par injections répétées pendant une période pouvant aller jusqu'à 90 jours.

19. Inhibiteur de SHP-2 pour utilisation dans la prévention et/ou le traitement d'une infection secondaire, dans laquelle l'inhibiteur de SHP-2 est choisi parmi un anticorps anti-SIRPα, un fragment d'anticorps anti-SIRPα, un anticorps recombinant anti-SIRPα, un peptide de liaison, un siRNA, un oligo antisens, un piège à ligands ou une petite molécule inhibiteur de SIRPα.

20. Inhibiteur de SHP-2 pour utilisation selon la revendication 19, dans lequel l'infection secondaire est choisie dans le groupe comprenant la pneumonie, l'infection pleurale, l'infection urinaire, l'infection péritonéale, l'abcès intra-abdominal, la méningite, l'infection médiastinale et l'infection des tissus mous ou de la peau.

21. Inhibiteur de SHP-2 pour utilisation selon les revendications 19 ou 20, dans laquelle l'inhibiteur est utilisé à des doses allant d'environ 0,01 à 2000 mg, de préférence d'environ 0,01 à 1000 mg.

22. Inhibiteur de SHP-2 pour utilisation selon l'une des revendications 19 à 21, dans lequel l'inhibiteur est administré en une seule injection ou en injections répétées pendant 90 jours au maximum.

23. Méthode ex *vivo* pour déterminer la susceptibilité d'un sujet à une maladie secondaire comprenant
a. Détermination de la concentration (Cₐ₁) et/ou du niveau d'expression (Lₐ₁) de SIRPα dans un échantillon biologique dudit sujet,
b. Comparaison de la concentration mesurée Cₐ₁ et/ou de l'expression Lₐ₁ avec une valeur de référence correspondante C_{aref} et/ou L_{aref} en calculant un score S5= Cₐ₁/C_{aref} et/ou S6= L ₐ₁/L_{aref},
c. Déterminer la susceptibilité à une maladie secondaire en fonction de la valeur du score S5 et/ou S6 obtenu : si S5 > 1 et/ou S6 > 1 , le sujet étant considéré comme susceptible de présenter un déficit de la réponse immunitaire vis-à-vis d'un agent pathogène quelconque, ou, si S5 ≤ 1 et/ou S6 ≤ 1, le sujet étant considéré comme peu susceptible de présenter un déficit de la réponse immunitaire vis-à-vis d'un agent pathogène quelconque.

24. Méthode selon la revendication 23, dans laquelle l'échantillon biologique est choisi dans le groupe constitué d'un échantillon de liquide trachéal, de lavages bronchoalvéolaires, de liquide pleural et/ou de leucocytes circulants.

25. Méthode selon l'une des revendications 23 à 24, dans laquelle la valeur de référence C_{aref} est comprise entre 1 pg/mL et 100 mg/mL.

26. Méthode selon l'une des revendications 23 à 25, dans laquelle la valeur de référence L_{aref} est de 0 à 100 ratio de gène de ménage (PCR) ou de 10 à 80% de cellules positives (cytométrie de flux).

27. Utilisation de SIRPα de surface comme biomarqueur d'une infection secondaire.
